# EUROPEAN PATENT APPLICATION

(11) **EP 3 653 208 A1**
(43) Date of publication of application: **20.05.2020**
(21) Application number: 18382809.4
(22) Date of filing: 15.11.2018
(51) Int. Cl.: A61K 31/41, A61K 31/4439, A61K 31/454, A61K 31/497, A61K 31/501, A61K 31/5355, A61K 31/55, A61P 35/00

(54) **COMPOUNDS THAT SELECTIVELY AND EFFECTIVELY INHIBIT HAKAI-MEDIATED UBIQUITINATION, AS ANTI-CANCER DRUGS**

(71) Applicant: Fundación Profesor Novoa Santos, 15006 A Coruna (ES); Universidad de Alcalá, 28801 Madrid (ES)
(72) Inventor: FIGUEROA CONDE-VALVÍS, Angélica, 15006 A Coruña (ES); GAGO BADENAS, Federico, 28801 Alcalá de Henares (ES); MARTÍNEZ IGLESIAS, Olaia Antía, 15006 A Coruña (ES); CARBALLO CASTOSA, Raquel, 15006 A Coruña (ES); CORTÉS CABRERA, Álvaro, 28801 Alcalá de Henares (ES); CASAS PAIS, Alba, 15006 A Coruña (ES)
(74) Representative: Hoffmann Eitle

(57) **Abstract**

The present invention provides a class of compounds, which includes enantiomers and pharmaceutically acceptable salts thereof, that selectively and effectively inhibit Hakai-mediated ubiquitination, preferably without affecting Hakai protein levels, thereby representing excellent anti-cancer drugs useful in the treatment of a variety of cancers, such as carcinomas, in particular, tumors arising from the epithelial layers of the gastrointestinal track including month (oral cancer), esophagus, stomach, and small and large intestines (such as rectal or colon cancer). It also includes skin cancer, mammary gland (breast cancer), pancreas cancer, lung cancer, head and neck cancer, liver cancer, ovary cancer, cervix cancer, uterus cancer, gallbladder cancer, penile cancer, and urinary bladder cancer (such as renal, prostate or bladder cancer).

## Description

### FIELD OF THE INVENTION

The present invention relates to a novel class of compounds and to compositions comprising the same. The compounds and compositions (such as pharmaceutical compositions) of the present invention can be used as medicaments in the treatment of cancer.

### STATE OF THE ART

Carcinoma, the most common type of cancer, arises from epithelial cells. The transition from adenoma to carcinoma is associated with the loss of E-cadherin and, in consequence, the disruption of cell-cell contacts. E-cadherin is a tumor suppressor, and it is down-regulated during epithelial-to-mesenchymal transition (EMT); indeed, its loss is a predictor of poor prognosis. Hakai is an E3 ubiquitin-ligase protein that mediates E-cadherin ubiquitination, endocytosis and finally degradation, leading the alterations of cell-cell contacts. Although E-cadherin is the most established substrate for Hakai activity, other regulated molecular targets for Hakai may be involved in cancer cell plasticity during tumor progression. In other works, the authors of the present invention have employed an iTRAQ approach to explore novel molecular pathways involved in Hakai-driven EMT during tumor progression. Their results show that Hakai may have an important influence on cytoskeleton-related proteins, extracellular exosome-associated proteins, RNA-related proteins and proteins involved in metabolism. Moreover, a profound decreased expression in several proteasome subunits during Hakai-driven EMT was highlighted. Since proteasome inhibitors are becoming increasingly used in cancer treatment, these findings suggest that the E3 ubiquitin-ligase, such as Hakai, may be a better target than proteasome for using novel specific inhibitors in tumor subtypes that follow EMT, such as carcinomas, tumors with mesenchymal phenotype or tumors where enhanced Hakai expression is detected respect to normal tissues. However, until now, compounds capable of effectively inhibiting Hakai-mediated ubiquitination that are especially suitable as therapeutic tools for the treatment of carcinomas have not been disclosed.

The present invention provides for such class of compounds, which includes enantiomers and pharmaceutically acceptable salts thereof, that selectively and effectively inhibit Hakai-mediated ubiquitination, preferably without affecting Hakai protein levels, and that at the same time represent excellent anti-cancer drugs useful in the treatment of a variety of cancers, such as carcinomas

### BRIEF DESCRIPTION OF THE INVENTION

The present invention provides a class of compounds, which includes enantiomers and pharmaceutically acceptable salts thereof, that selectively and effectively inhibit Hakai-mediated ubiquitination, preferably without affecting Hakai protein levels, thereby representing excellent anti-cancer drugs useful in the treatment of a variety of cancers, such as carcinomas, in particular, tumors arising from the epithelial layers of the gastrointestinal track including month (oral cancer), esophagus, stomach, and small and large intestines (such as rectal or colon cancer). It also includes skin cancer, mammary gland (breast cancer), pancreas cancer, lung cancer, head and neck cancer, liver cancer, ovary cancer, cervix cancer, uterus cancer, gallbladde cancer, penile cancer, and urinary bladder cancer (such as renal, prostate or bladder cancer). The compounds of the present invention also show lower toxicity which renders the present compounds very attractive. Such compounds are represented by formula (I) below.

Therefore, a first aspect of the present invention, refers to a compound of formula (I) 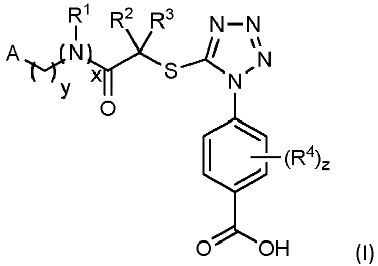 wherein:
- A represents a group selected from aryl, heteroaryl and cyclic amides optionally substituted by 1 or 2 groups that are independently selected from:
   a) halogen atom, -CN, -N(R^{a})R^{b}, -OR^{a}, -C(=O)R^{a}, -C(=O)OR^{a}, -C(=O)N(R^{a})R^{b}, -OC(=O)-R^{a}, -N(R^{c})C(=O)R^{b}, -NR^{c}SO₂R^{a}, -SO₂N(R^{a})R^{b}, -SR^{a}, -S(O)R^{a}, -S(O)₂R^{a};
   b) linear or branched C₁-C₆ alkyl, optionally substituted by 1, 2 or 3 halogen atoms;
   c) C₃-C₆ cycloalkyl which optionally contains 1 or 2 heteroatoms selected from O, S and N, and which ring is optionally substituted by C₁-C₃ alkyl;
   d) phenyl or C₅-C₆ heteroaryl each optionally substituted by halogen atom, cyano group, C₁-C₃ alkyl or cyclopropyl;
- each R^{a}, R^{b} and R^{c} independently represents:
   a) hydrogen atom,
   b) linear or branched C₁-C₁₂ alkyl, C₃-C₆ cycloalkyl and C₄-C₆ heterocycloalkyl, which are optionally substituted by 1, 2 or 3 substituents selected from a carbonyl group, halogen atom, hydroxy, phenyl, C₃-C₆ cycloalkyl, linear or branched C₁-C₆ alkoxy, amino, alkylamino, dialkylamino, linear or branched C₁-C₆ alkylcarbonyl,
   c) phenyl or C₅-C₆ heteroaryl group, which are optionally substituted by 1, 2 or 3 substituents selected from halogen atom, cyano group, linear or branched C₁-C₆ alkyl, linear or branched C₁-C₆ haloalkyl, hydroxy, linear or branched C1-C6 alkoxy, amino, alkylamino, dialkylamino;
   d) R^{a} and R^{b} form together with the nitrogen atom to which they are attached, a 3- to 8 membered ring which optionally contains a further heteroatom selected from O, N and S, and which ring is optionally substituted by 1, 2 or 3 substituents selected from carbonyl group, linear or branched C₁-C₆ alkyl, linear or branched C₁-C₆ haloalkyl, linear or branched C₁-C₆ alkylcarbonyl;
- x and y are integers independently selected from 0 and 1;
- R¹ represents a group selected from hydrogen, cyclopropyl or linear or branched C₁-C₆ alkyl, wherein said alkyl is optionally substituted by 1, 2 or 3 halogen atoms;
- when y is 0, then 2 or 3 carbon atoms of R¹ can form a 5- or 6-membered ring together with the neighboring nitrogen atom and the 2 adjacent carbon atoms of the aromatic ring to which the nitrogen is attached;
- each R² and R³ independently represent a group selected from hydrogen, cyclopropyl or linear or branched C₁-C₆ alkyl;
- R² and R³ can form a 3-or 4-membered spiro ring together with the carbon atom to which they are both attached;
- z is an integer selected from 0, 1, 2 or 3;
- R⁴ represents a group selected from -CN, cyclopropyl or linear or branched C₁-C₆ alkyl, said alkyl is optionally substituted by 1, 2 or 3 halogen atoms; wherein the group R⁴, if present, replaces the hydrogen atom of one of the groups CH present in the phenyl ring to which R⁴ is attached;
and pharmaceutically acceptable salts thereof;
for use in the treatment of cancer, in particular for use in the treatment of carcinoma, more particularly for use in the treatment of carcinomas, which include tumors arising from the epithelial layers of the gastrointestinal track including month (oral cancer), esophagus, stomach, and small and large intestines (such as rectal or colon cancer). It also includes skin cancer, mammary gland (breast cancer), pancreas cancer, lung cancer, head and neck cancer, liver cancer, ovary cancer, cervix cancer, uterus cancer, gallbladder cancer, penile cancer, and urinary bladder cancer (such as renal, prostate or bladder cancer).

It is herein noted, that in the context of the present invention, the term "carcinoma" is understood as a type of cancer arising in the epithelial tissues. These cover the outside of the body, as the skin and also cover and line all the organs inside the body, such as the organs of the digestive system. Furthermore, they line the body cavities, such as the inside of the chest cavity and the abdominal cavity. Carcinomas are the most common type of cancer. These tumors are responsible for more than 80% of the cancer-related deaths in the Western world.

In a preferred embodiment of the first aspect of the invention, each R² and R³ independently represents a group selected from cyclopropyl or linear or branched C₁-C₆ alkyl.

In another preferred embodiment of the first aspect of the invention or of any of its preferred embodiments, R² and R³ form a 3-or 4-membered spiro ring together with the carbon atom to which they are both attached.

In another preferred embodiment of the first aspect of the invention or of any of its preferred embodiments, z is an integer selected from 1, 2 or 3; wherein R⁴ represents a group selected from -CN, cyclopropyl or linear or branched C₁-C₆ alkyl, wherein said alkyl is optionally substituted by 1, 2 or 3 halogen atoms; and wherein group R⁴ replaces the hydrogen atom of one of the groups CH present in the phenyl ring to which R⁴ is attached.

In another preferred embodiment of the first aspect of the invention or of any of its preferred embodiments, one or both of the integers x and y equal 1 and A represents a group selected from aryl, heteroaryl and cyclic amides optionally substituted by 1 or 2 groups that are independently selected from halogen atom, -CN, -N(Ra)Rb, -ORa, -C(=O)Ra, -C(=O)ORa, -C(=O)N(Ra)Rb, -OC(=O)-Ra, -N(Rc)C(=O)Rb, -NRcSO2Ra, -SO2N(Ra)Rb, -SRa, -S(O)Ra, -S(O)2Ra, linear or branched C1-C6 alkyl, wherein said alkyl is optionally substituted by 1, 2 or 3 halogen atoms; C3-C6 cycloalkyl which optionally contains 1 or 2 heteroatoms selected from O, S and N, and which ring is optionally substituted by C1-C3 alkyl; phenyl or C5-C6 heteroaryl each optionally substituted by halogen atom, C1-C3 alkyl or cyclopropyl.

In yet another preferred embodiment of the first aspect of the invention or of any of its preferred embodiments, the compound is selected from the list consisting of any of the following compounds:

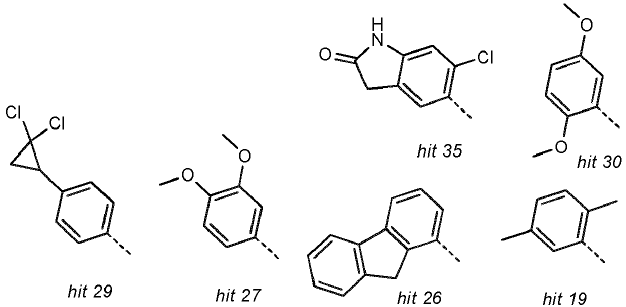

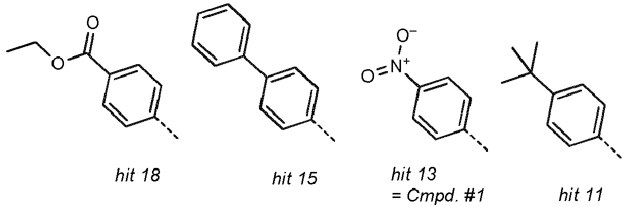

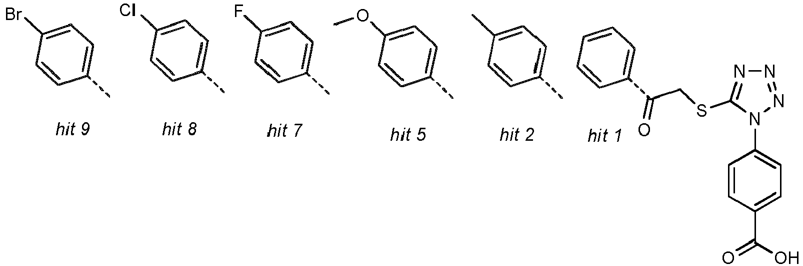

More preferably, the compound is

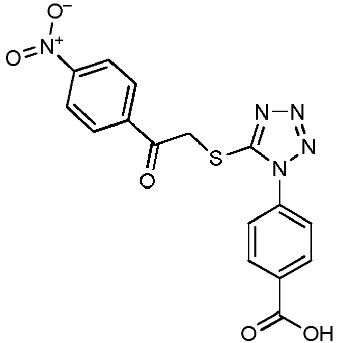

### 4-(5-((2-(4-nitrophenyl)-2-oxoethyl)thio)-1H-tetrazol-1-yl)benzoic acid

Still more preferably, both of the integers x and y equal 0 and A represents a benzyl substituted by 1 group, preferably at the para-position, selected from halogen from a halogen atom, -ORa, - -OC(=O)-Ra, -N(Rc)C(=O)Rb, -NRcSO2Ra, -SO2N(Ra)Rb, -SRa, -S(O)Ra, or -S(O)2Ra. Preferably, said group is selected from a halogen atom, or -ORa. Preferably said compounds are selected from the group consisting of hits 5 to 9 above. More preferably, said compound is hit 7.

In yet another preferred embodiment of the first aspect of the invention or of any of its preferred embodiments, the compound is a ketoheteroaryl, preferably selected from the list consisting of any of the following compounds:

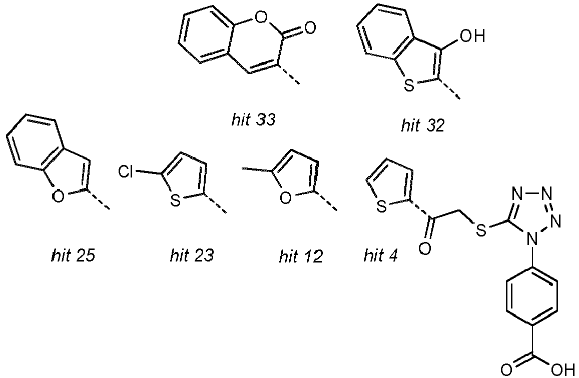

Or

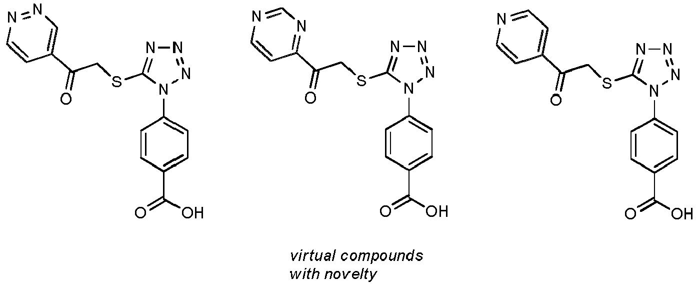

More preferably, a still more preferred embodiment of the present invention refers to any of the ketoheteroaryls compounds useful to practice the present invention as illustrated through-out the present specification. In particular, preferably, both of the integers x and y equal 0 and A represents a heteroaryl optionally substituted by a halogen atom or -ORa. Preferably, said compound is selected from the group consisting of hit 23 or hit 25 above.

In yet another preferred embodiment of the first aspect of the invention or of any of its preferred embodiments, the compound is a cyclic amide, preferably selected from the list consisting of any of the following compounds:

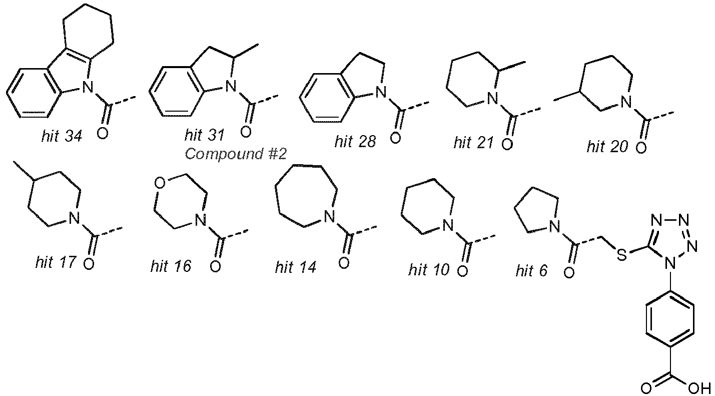

Preferably, the compound is the substituted indoline (and indole) analog illustrated below:

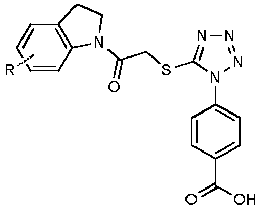

Wherein R represents a group selected from hydrogen, cyclopropyl or linear or branched C1-C6 alkyl, wherein said alkyl is optionally substituted by 1, 2 or 3 halogen atoms. More preferably, R is a methyl group. Preferably, said compound is selected from the group consisting of hit 10 or hit 16 above.

In yet another preferred embodiment of the first aspect of the invention or of any of its preferred embodiments, the compound is benzylamide, preferably selected from the list consisting of any of the following compounds:

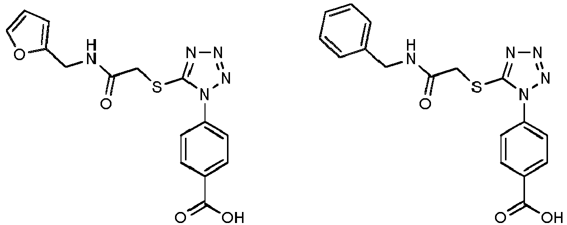

Further compounds useful in the present invention are illustrated through-out the present specification.

### BRIEF DESCRIPTION OF THE FIGURES

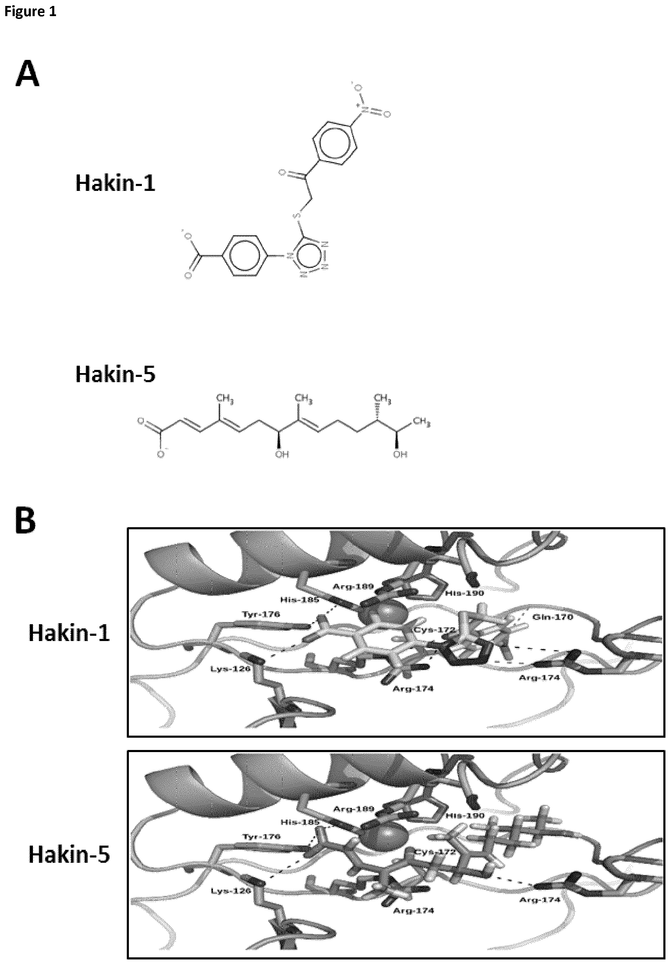

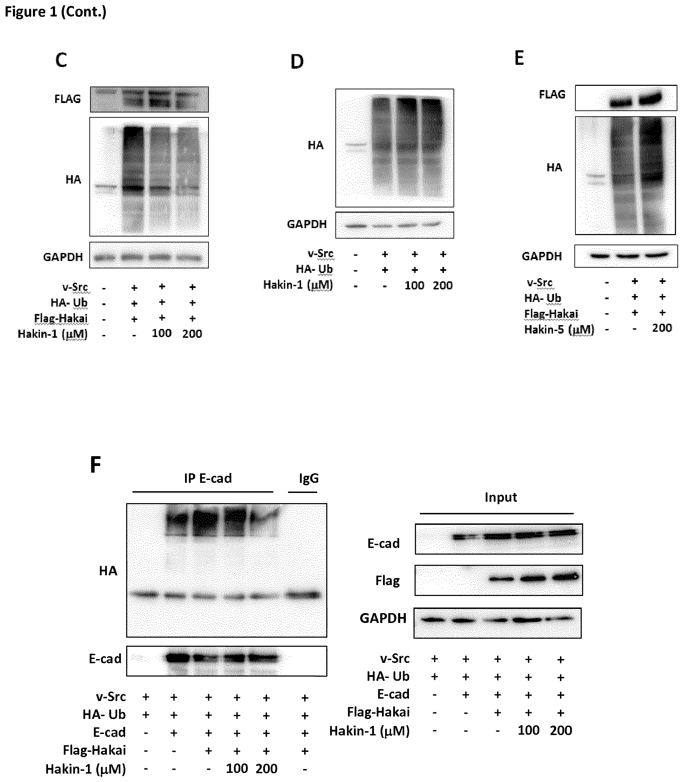

**Figure 1****. *In silico* and *in vitro* screen for E3 ubiquitin-ligase Hakai inhibitors (A)** Chemical structure of Hakin-1 and Hakin-5. **(B)** Predicted binding poses for Hakin-1 (left panel, in yellow) and Hakin-5 (right panel, in orange) molecules *docked* within Hakai dimers (represented in blue and green), as determined by the CRDOCK docking program. **(C)** *In vivo* Hakai-dependent ubiquitination assay in 293T cells transfected with Flag-Hakai, v-Src and HA-ubiquitin in presence of either DMSO or compound Hakin-1. **(D)** *In vivo* Hakai-dependent ubiquitination assay in 293T cells transfected with Flag-Hakai, v-Src and HA-ubiquitin in the presence of DMSO or compound Hakin-5. **(E)** *In vivo* ubiquitination assay in 293T cells transfected with v-Src and HA-ubiquitin in the presence of DMSO or compound Hakin-1. **(F)** Effect of Hakin-1 on the Hakai-dependent ubiquitination of the E-cadherin complex. pcDNA-Flag-Hakai, pcDNA-myc-E-cadherin, pSG-v-Src and pBSSR-HA-ubiquitin were transiently transfected into 293T cells. Immunoprecipitation was performed with the anti-E-cadherin antibody before western blotting using the indicated antibodies.

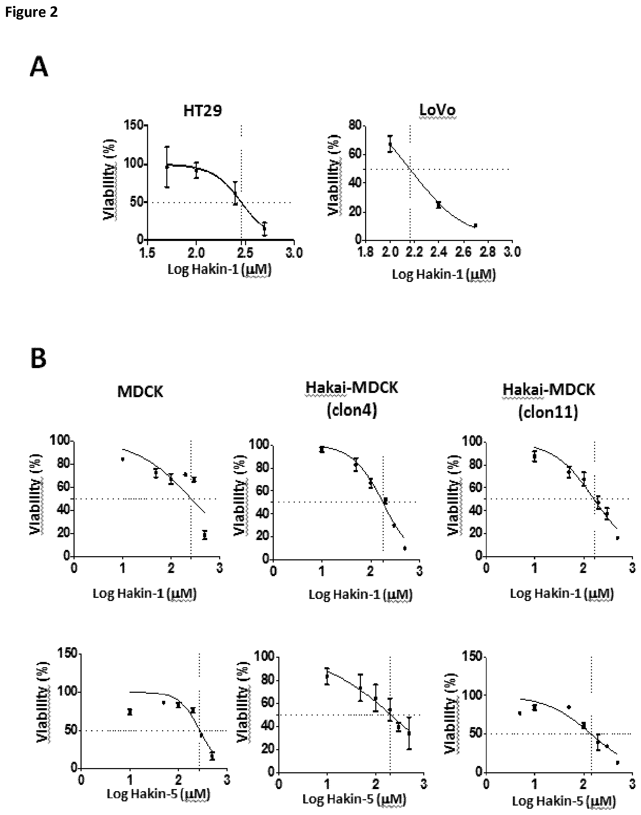

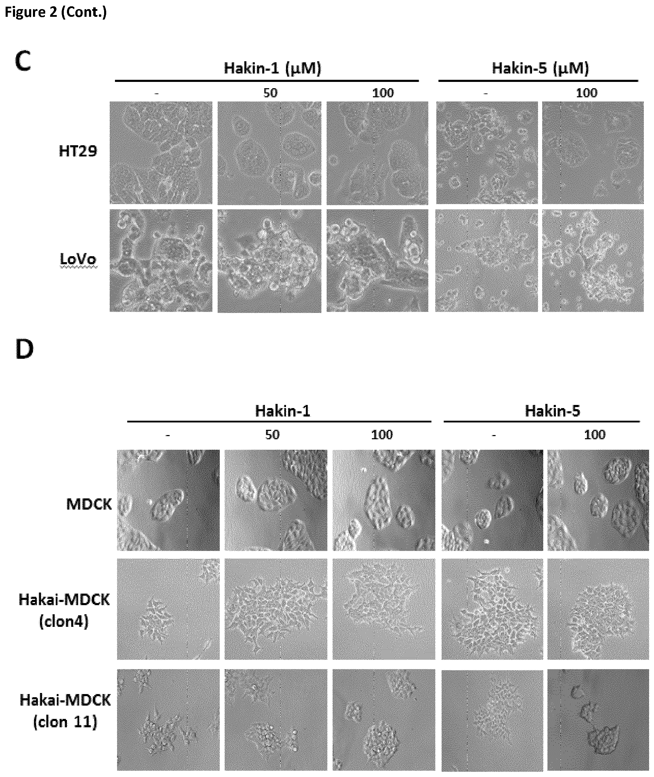

**Figure 2****. Hakin-1 induces cytotoxicity and an epithelial phenotype on epithelial tumour cell lines. (A)** HT29 and LoVo cells were treated an increasing range of concentrations of Hakin-1 or Hakin-5 and cell viability was measured by MTT assay. Assay was performed in 6 replicates and represented as mean ± SD of three independent experiments. **(B)** Cell viability was measured as indicated in (A) for MDCK, Hakai-MDCK cell lines (clone 4 and clone 11) using Hakin-1 (upper panel) or Hakin-5 (bottom panel). **(C-D)** Phase-contrast images of HT29 and LoVo cell lines (C) and MDCK, Hakai-MDCK cell lines, clone 4 and clone 11 (D) under Hakin-1 or Hakin-5 treatment. Images were obtained using a 20X objective.

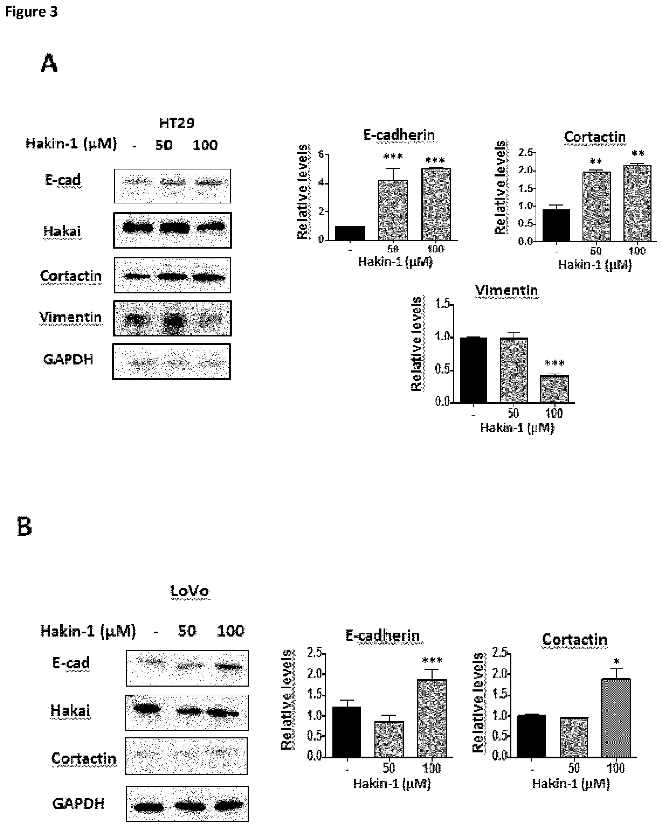

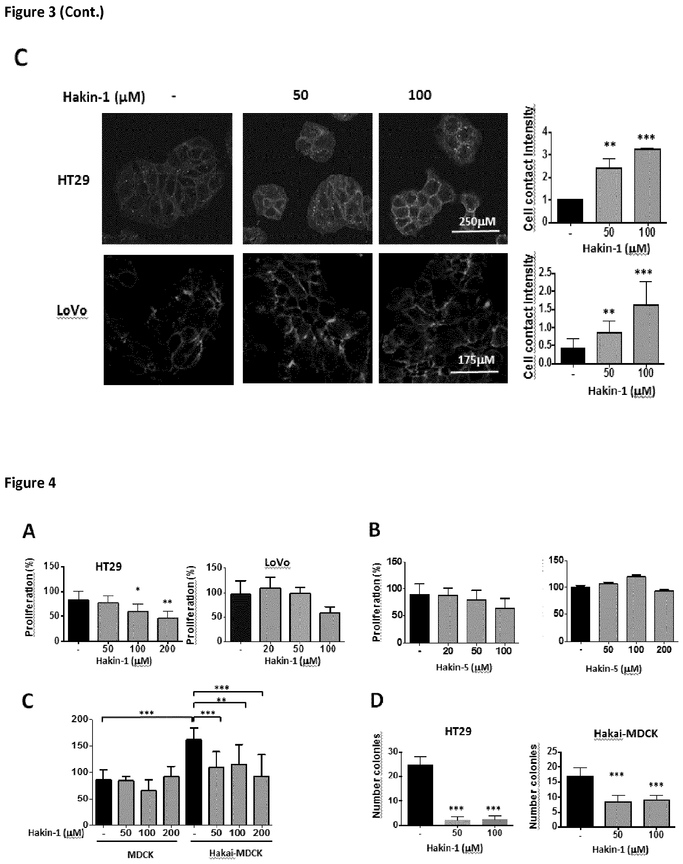

**Figure 3****. Hakin-1 induces mesenchymal-to-epithelial transition in epithelial tumour cell lines. (A)** Western blotting analyses of EMT markers upon Hakin-1 treatment in HT-29 cell line (left panel), and quantification by densitometry is shown (right panel). **(B)** Western blotting analyses of EMT markers upon Hakin-1 treatment in LoVo cells (left panel), and quantification by densitometry is shown (right panel). GAPDH was used as loading control. Quantifications were performed as indicated in Materials and Methods and the western blotting data are representative of three experiments. Data shows the average of three independent experiments and are represented as mean ± SD (*p < 0.05; **p < 0.01; ***p < 0.001). **(C)** Immunofluorescence of E cadherin in HT-29 and LoVo cell lines in presence of DMSO or Hakin-1 treatment after 48h. Images were obtained with a 40x objective. Quantification was performed with Image J programme and results are expressed as mean ± SD of three independent different experiments (**p < 0.01; *** p < 0.001). Scale bar, 250 µM for HT29 cells and 175 µµM for LoVo cells.
**Figure 4****. Antiproliferative and antioncogenic effect of Hakin-1 in tumour epithelial cells. (A)** HT29 and LoVo cells were treated with Hakin-1 for 48h and proliferation was measured by a BrdU assay as indicated in Material and Methods. Results are expressed as mean ± SD of eight replicates and experiments were repeated three times (*p < 0.05; ** p < 0.01; *** p < 0.001).. **(B)** HT29 and LoVo cells were treated with Hakin-5 for 48h and proliferation was measured as indicated in A **(C)** MDCK and Hakai-MDCK cells were treated with increasing concentrations of Hakin-1 for 48h and proliferation was measured as indicated in A. **(C)** Soft agar assay in HT29 (left panel) and Hakai-MDCK (right panel) cell lines. Colonies grew for 28 days (HT29) or 21 days (Hakai-MDCK) and were counted as indicated in Materials and Methods. Quantification of the colonies was performed in triplicates and represented as mean ± SD of three independent experiments (**p < 0.01; *** p < 0.001).

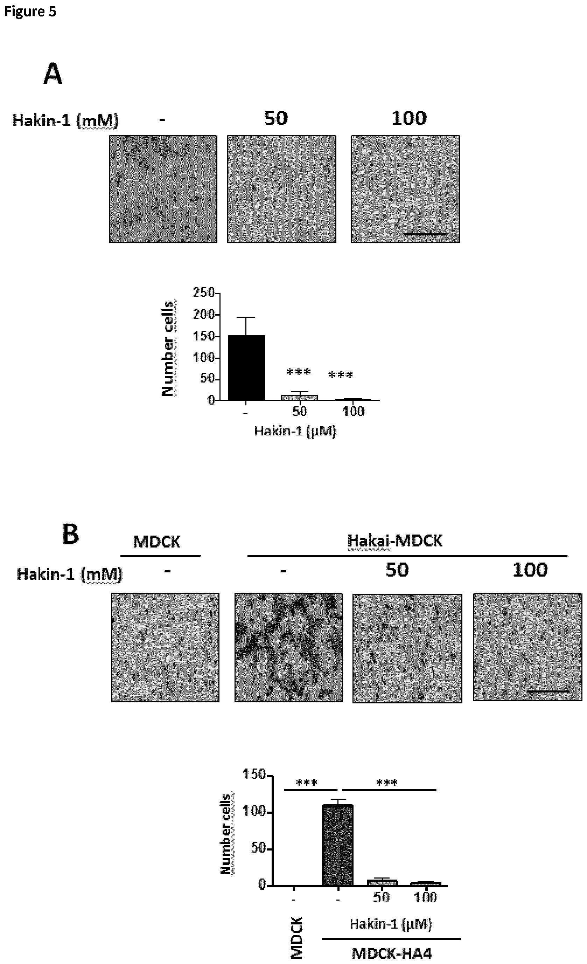

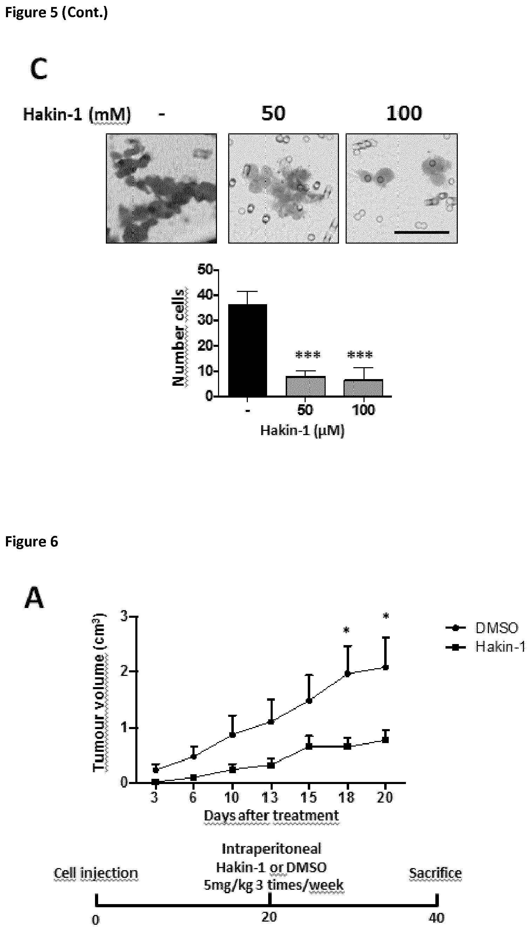

**Figure 5****. Hakin-1 reduces cell invasion and cell migration of epithelial tumour cells. (A)** Invasion assay in LoVo cell line was performed as described in Materials and Methods. Cells were treated in presence of DMSO or Hakin-1 for 48h before being seeded into an invasion chamber. Representative images were taken using the 20X objective (upper panel) and quantification of the photographed invasive cells are shown (bottom panel). **(B)** Invasion assay was performed as indicated in A by using MDCK and Hakai-MDCK cells. **(C)** Migration assay in HT29 cells was analysed after treatment with DMS or Hakin-1 during 48 h. Cells were seeded in a migration chamber as described in Materials and Methods. Representative images are shown (upper panel) and quantification of migrating cells is shown (bottom panel). Results are represented as mean ± SD of triplicates of three independent experiments (***p < 0.001).

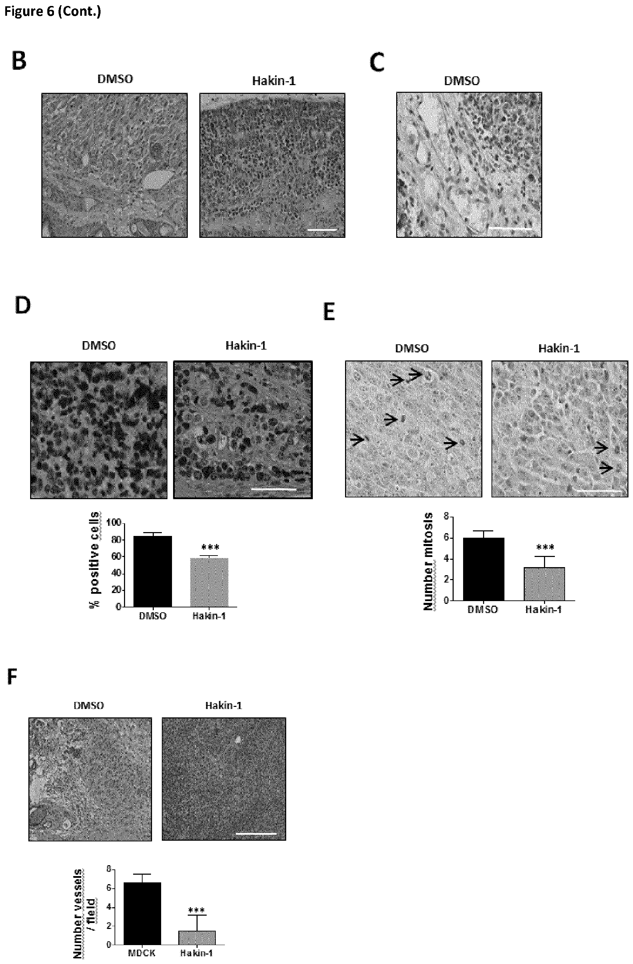

**Figure 6****. Hakin-1 inhibits tumour growth in xenografted mice. (A)** Effects of Hakin-1 on tumour growth in nude mice inoculated into the flank with Hakai-MDCK cells (n=6 tumours). Tumour growth curve is shown in upper panel. Error bars represented the mean ± SEM (*p < 0.05). A schematic representation of the experiment design is shown in the bottom panel. **(B)** H&E staining of Hakai-MDCK tumours at the end point treated with DMSO (left panel) or Hakin-1 (right panel). Images were obtained with a 20x objective. Scale bar, 300 µM. **(C)** H&E staining showing the infiltration of a blood vessel by tumour cells. Images were obtained with a 20x objective. Scale bar, 500 µM. **(D)** Immunohistochemistry of Ki67 marker in Hakai-MDCK tumours treated with DMSO (left panel) or Hakin-1 (right panel). Representative images were obtained with 40x objective. Scale bar, 500 µM. Quantification of the percentage of positive cells is shown in the bottom panel. **(E)** Representative image of Hakai-MDCK tumour in nude mice stained with H&E is shown. Pictures were obtained with a 40x objective (upper panel). Quantification of the number of mitosis in high magnification field is shown (bottom panel). Scale bar, 500 µM. **(F)** Immunohistochemistry of CD31 marker in Hakai-MDCK tumour of injected nude mice treated with DMSO (left panel) or Hakin-1 (right panel). Images were obtained with a 20x objective. Scale bar, 500 µM. Quantification of the number of fields is shown in the bottom panel and is expressed as mean ± SEM (***p < 0.001).

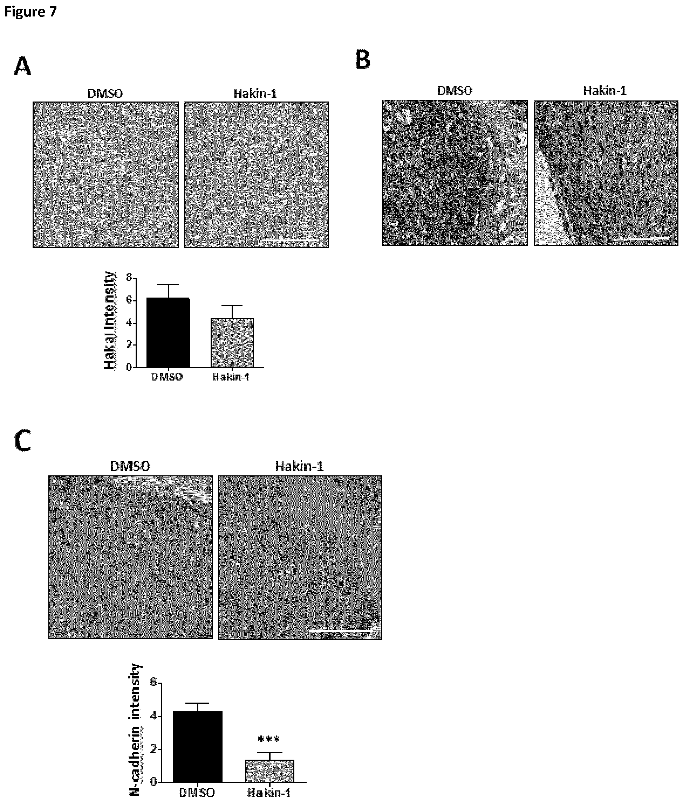

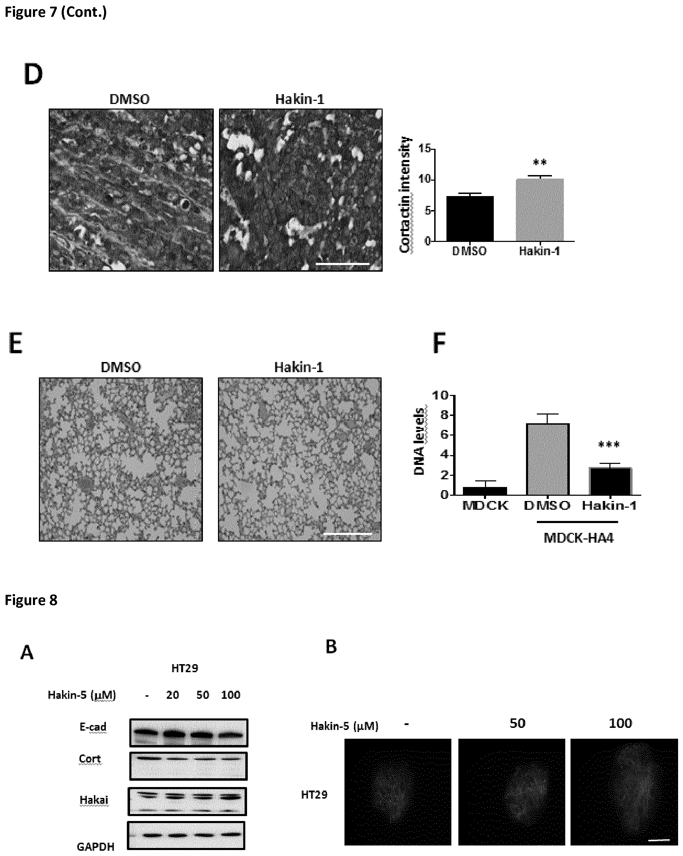

**Figure 7****. Hakin-1 treatment reduces mesenchymal markers of tumours xenograft and micrometastasis formation in lung of nude mice. (A-C)** Immunohistochemical staining for Hakai (A), E-cadherin (B) and N-cadherin (C). Representative images were obtained with a 20x objective (upper panel). Quantification of significantly protein expression intensity is shown in bottom panel. **(D)** Immunohistochemical staining for Cortactin antibody and protein expression quantification is shown (upper and lower panels, respectively). Images were obtained with 40x objective. **(E)** H&E staining of mice lungs. Representative images were obtained with a 10x objective. **(F)** Real-time quantitative PCR using primers for HA epitope and Hakai to detect the presence of DNA of Hakai-MDCK cells into the mice lung. Results are expressed as mean ± SEM (***p < 0.001). Scale bar, 500 µM.
**Figure 8****. Hakin-5 does not affect the EMT markers expression. (A)** Western blotting of E-Cadherin, Cortactin and Hakai in HT29 cells after Hakin-5 treatment for 48h with. **(B)** Immunofluorescence of E-Cadherin in HT29 cells treated with Hakin-5 for 48h. Images were taken with the 40x objective. Scale bar, 250 µM.

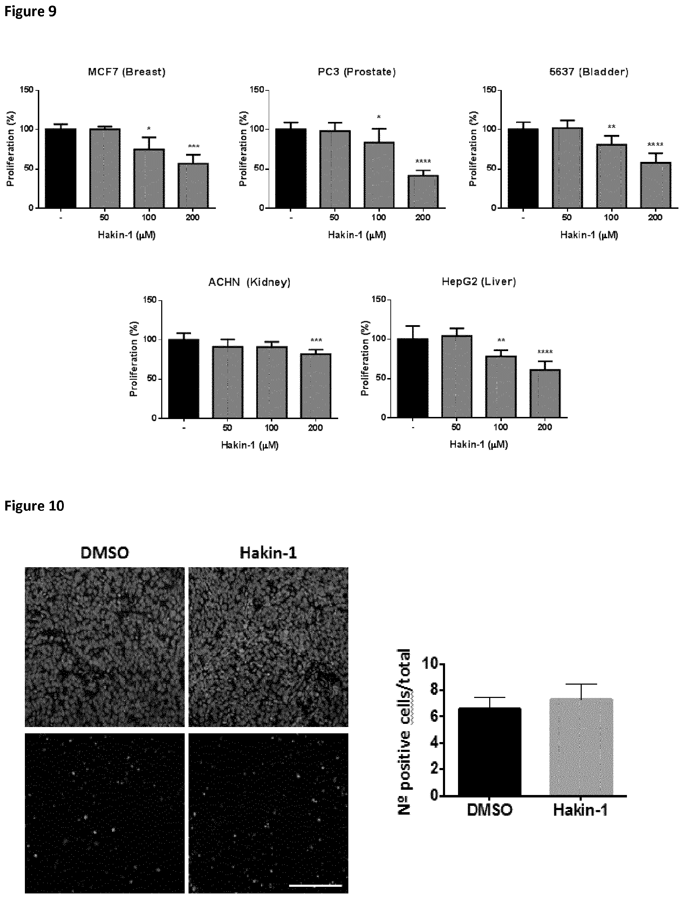

**Figure 9****. Effect of Hakin-1 in human cancer cells.** Breast cancer MCF7 cell, prostate cancer PC-3 cells, bladder cancer 5637 cells liver, renal cancer ACHN and cancer liver cancer HepG2 cells were treated with Hakin-1 for 48h and proliferation was measured by a BrdU assay as indicated in Material and Methods. Results are expressed as mean ± SD of eight replicates and experiments were repeated three times (*p < 0.05; **p < 0.01; ***p < 0.001).
**Figure 10****. Hakin-1 does not affect cell apoptosis *in vivo* in xenograft mouse models.** Tunel assay was performed as indicated in Materials and Methods. A representative image is shown (left panel) and quantification of the number of positive cells is also represented (right panel) as mean ± SEM. Images were taken with 20x objective. Scale bar, 125 µM.

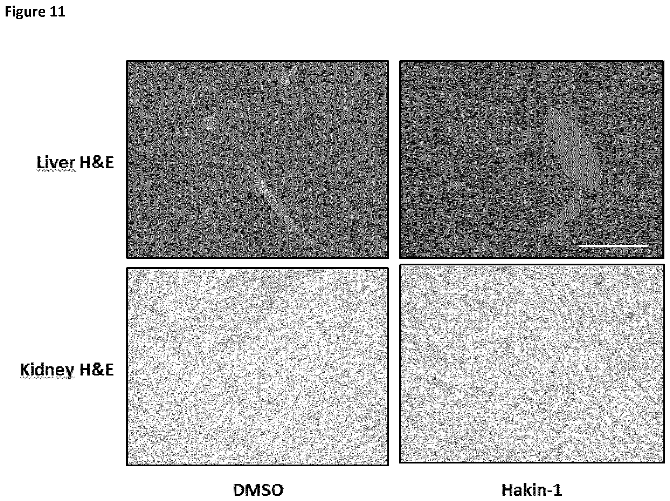

**Figure 11****. Intact cell morphology and tissue structure of liver and kidney *in vivo* in xenograft mouse models treated with Hakin-1.** H&E staining of liver (upper panel) and kidney (bottom panel) of nude mice treated with 5mg/kg of DMSO or Hakin-1. Images were taken with a 10x objective. Scale bar, 500 µM.

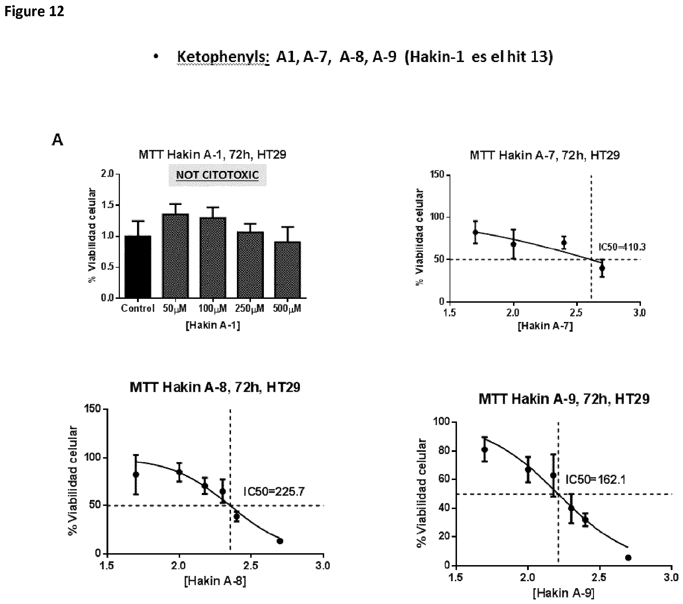

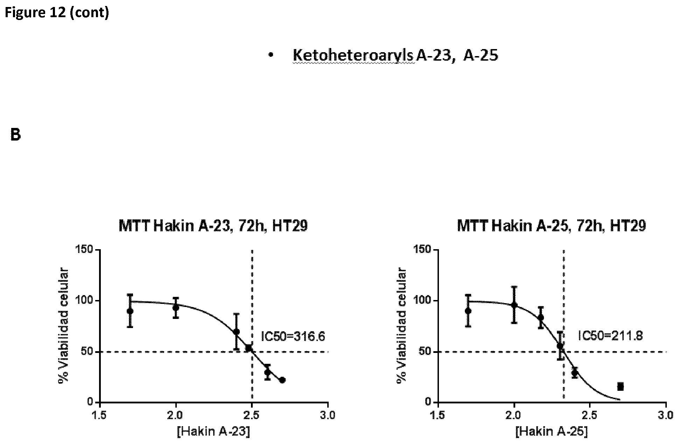

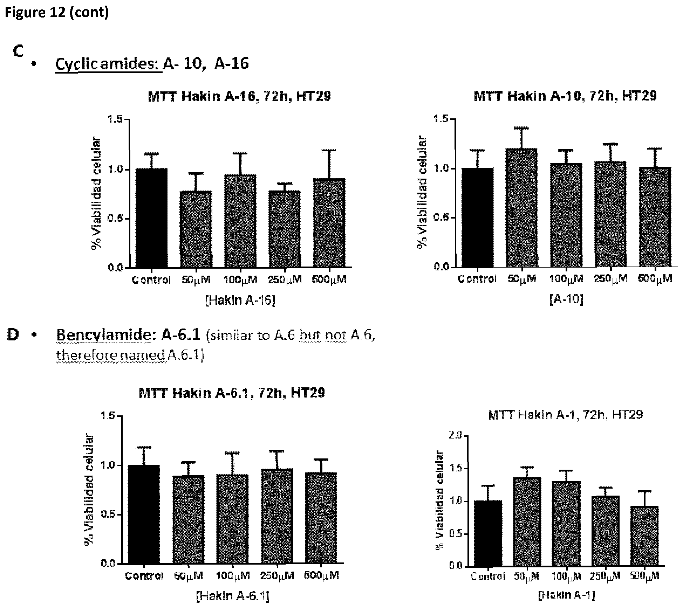

**Figure 12****. Effect of selected analogues on the cytotoxicity in HT29 epithelial tumour cell lines.** Cells were treated with increasing range of concentrations (50 µM, 100 µM, 250 µM and 500 µM) of **(A)** Ketophenyls A-1, A-7, A-8 and A-9 **(B)** ketoheteroaryls: A-23 and A-25 **(C)** Cyclic amides: A-10 and A-16 and **(D)** Bencylamide A-6.1. Cell viability was measured by MTT assay. Assays were performed using six replicates and represented as mean ± SD of two independent experiments.

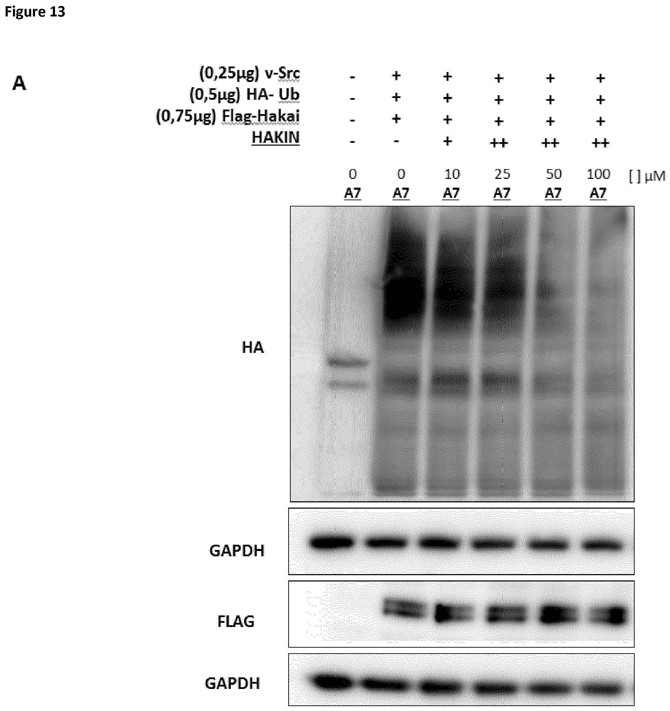

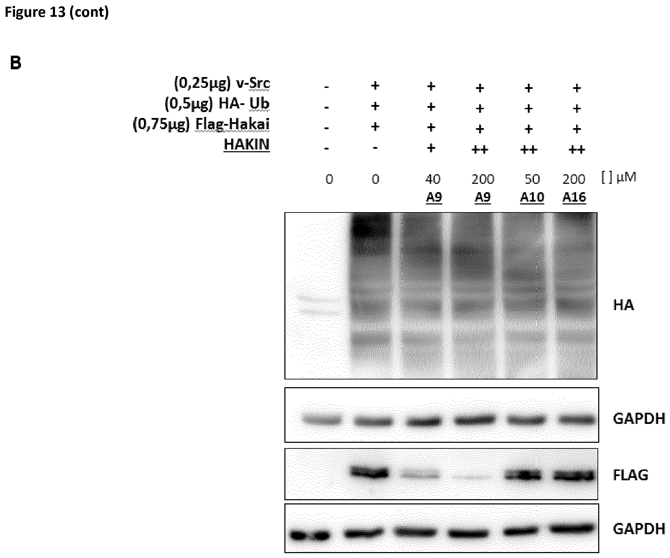

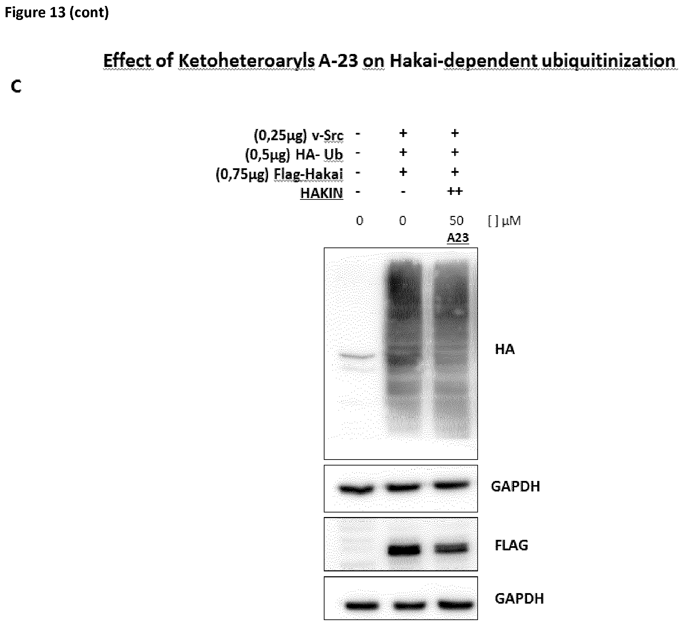

**Figure 13****. Effect of analogues inhibitors on Hakai-dependent ubiquitination.** *In vivo* Hakai-dependent ubiquitination assay in 293T cells transfected with Flag-Hakai, v-Src and HA-ubiquitin in the presence of DMSO or selected analogues. **(A)** Ketophenyls A-7 and A-9 **(B)** Ketophenyls A-9 and Cyclic amides: A-10 and A-16 and **(C)** ketoheteroaryls: A-23.

### Description of the invention

The present invention identifies 4-Tetrazolylbenzoic acids Hakin-1, Hakin-2 and Hakin-6 (from hereinafter compounds #1, 2 and 6) 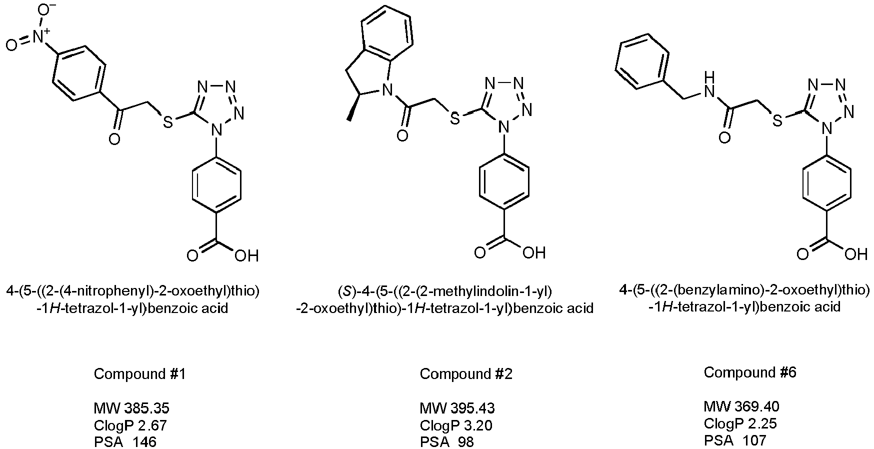 as inhibitors of Hakai capable of competing for the HYB binding site that is only present in the Hakai dimer. Hakai has been reported to be involved in tumor progression; therefore inhibitors of the interaction between Hakai and E-cadherin might be useful for the treatment of cancer. In this sense and as shown in the examples, inhibition of tumor progression has been herein demonstrated *in vitro* and *in vivo* utilizing compound #1. Analogs #2 and 6 have not been available for testing, but they are structurally closely related to compound #1 and the present description makes it plausible that these compounds also inhibit tumor progression.

Therefore, the present invention solves the technical problem of providing compounds having excellent anti-oncogenic effects and low toxicity. Thus, the compounds of the present invention can advantageously be used as a medicament and, particularly, in the treatment of a variety of cancers, such as carcinomas, in particular the gastrointestinal track cancer including month (oral cancer), esophagus, stomach, and small and large intestines (such as rectal or colon cancer). It also includes skin cancer, mammary gland (breast cancer), pancreas cancer, lung cancer, head and neck cancer, liver cancer, ovary cancer, cervix cancer, uterus cancer, gallbladder cancer, penile cancer, and urinary bladder cancer (such as renal, prostate or bladder cancer. Indeed, collectively, our data, as illustrated in the examples, show that Hakin-1 is a specific inhibitor for Hakai-mediated ubiquitination, without affecting Hakai protein levels (Example 1). In addition, Hakin-1 was able to suppress proliferation in Hakai-MDCK cell while no effect was detected in MDCK cells (Fig. 4c). Hakin-1 also inhibits cell proliferation in other epithelial cells lines such as breast cancer MCF7 cell, prostate cancer PC-3 cells, bladder cancer 5637 cells, liver cancer HepG2 cells and renal cancer ACHN cells. All these findings support an antitumor effect of Hakin-1 by acting on cell proliferation, oncogenic potential, cell motility and invasion. Hakin-1 treatment inhibits tumor growth in nude mice apparently without systemic toxicity (Fig. 11). Furthermore, Hakin-1 caused a significant reduction of micrometastasis detected in lung of the Hakai-MDCK xenograft mice compared to the control DMSO treated mice, while no detection was found in lung of non-transformed MDCK-injected mice (Fig. 7f). This result underscore that Hakin-1 inhibits the metastasis to lung in vivo.

Compound #1 (as already stated, also referred to as Hakin-1) is a 1,5-disubstituted tetrazole, a chemically and metabolically stable pharmacophore fragment frequently used in drug development (Tetrazole Derivatives as Promising Anticancer Agents, E. A. Popova et al., Anticancer Agents Med Chem. 2017 Mar 27. doi: 10.2174/1871520617666170327143148, Epub ahead of print). The tetrazole ring is substituted with a 4-carboxyphenyl group in position 1. In position 5, it is connected via a mercaptomethylcarbonyl linker with another phenyl ring.

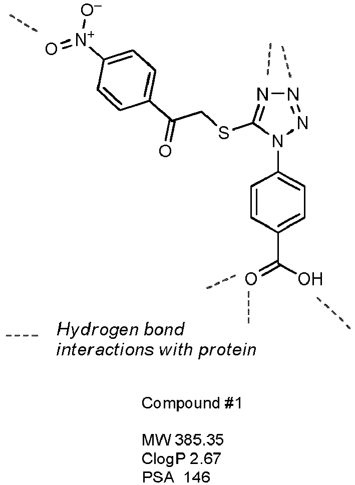

Important physico-chemical parameters like molecular weight, calculated lipophilicity logP and polar surface area are in the desired range for orally absorbed drugs that are unlikely to pass the blood-brain barrier. According to a snapshot from a docking study with compound 1 in the Hakai HYB site, the carboxyphenyl moiety makes 3 important hydrogen bond interactions with the protein and is likely to mimick the binding mode of the phosphotyrosine subtrates. Further hydrogen bonds are formed by two of the tetrazole nitrogen atoms which provide additional stability and hold the tetrazole-carboxyphenyl unit on both ends in a defined binding mode. Therefore, the following subunit seems to be essential and shall be considered as the core structure for identifying derivatives of compound #1:

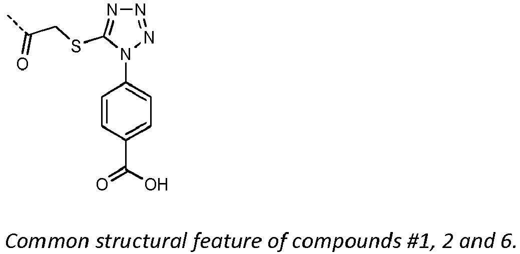

Departing from such common structural core, a number of modifications in order to optimize the activity (and other properties) of these compounds and provide analogs of these compounds, can be made. In this sense, there are several opportunities of introducing modifications in different parts of the molecule that represent an important objective in the optimization process and in the provision of analogs of compounds #1, 2 and 6. In this sense, a medicinal chemistry program would start focusing on the chemical space around compound #1 and with less priority around compounds #2 and #6. With this in mind we herein provide a group of different analogs of compounds #1, #2 and #6, useful in the present invention. Notably, useful as medicaments and, particularly, in the treatment of a variety of cancers, such as carcinomas, in particular tumors arising from the epithelial layers of the gastrointestinal track including month (oral cancer), esophagus, stomach, and small and large intestines (such as rectal or colon cancer). It also includes skin cancer, mammary gland (breast cancer), pancreas cancer, lung cancer, head and neck cancer, liver cancer, ovary cancer, cervix cancer, uterus cancer, gallbladder cancer, penile cancer, and urinary bladder cancer (such as renal, prostate or bladder cancer).

A first class of analogs is provided by connecting the core structure to a ring structure (Cy = any cycle) in order to find those analogs that are similar to compounds #1 and #2.

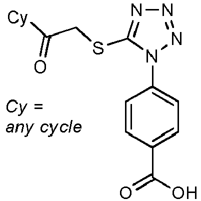

These types of compounds are grouped below according to the following structural subclasses a) to c).

### a) Ketophenyls (including compound #1):

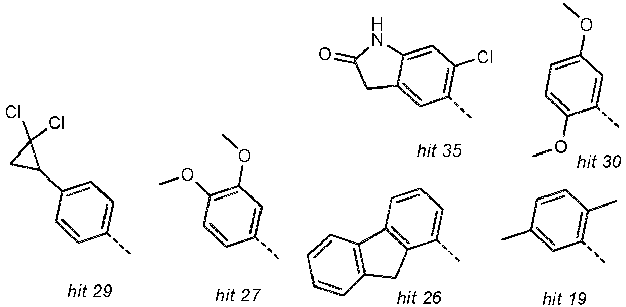

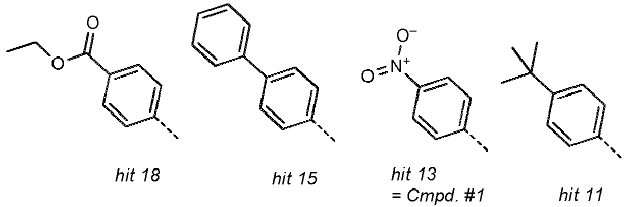

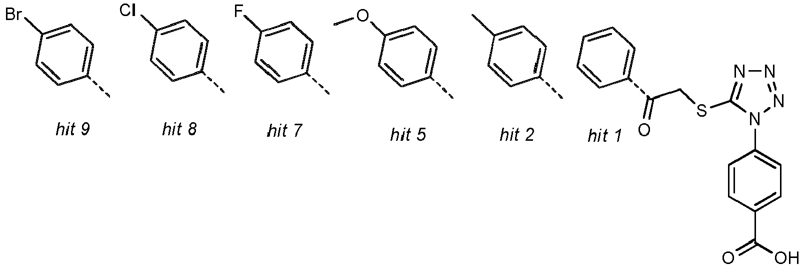

It is noted that these structures can comprise a substituent on the phenyl ring or in another part of the molecule, e.g. on the carboxyphenyl ring or on the carbon atom between the sulfur and the carbonyl group. The latter case has been exemplified as follows:

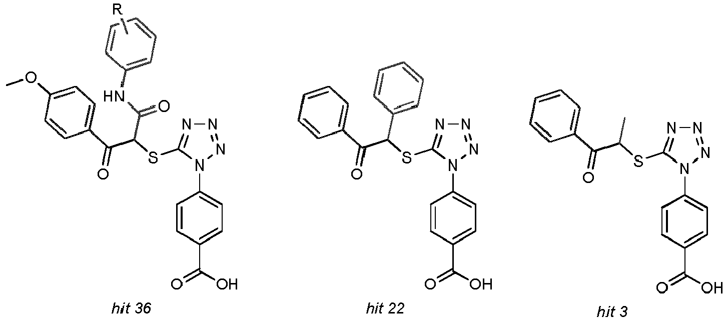

Moreover, the following examples, illustrate the introduction of modifications such as a cyclopropyl bridge or the addition of substituents on the carboxyphenyl ring:

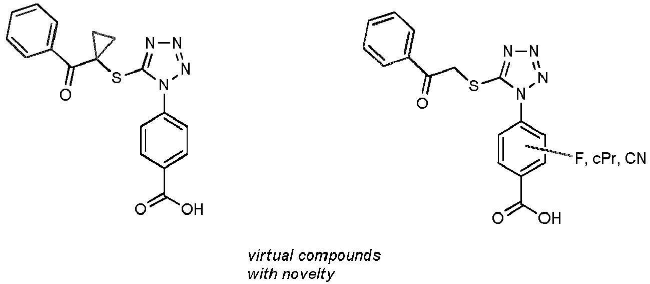

Particular examples of Ketophenyl compounds useful to practice the present invention are illustrated below:

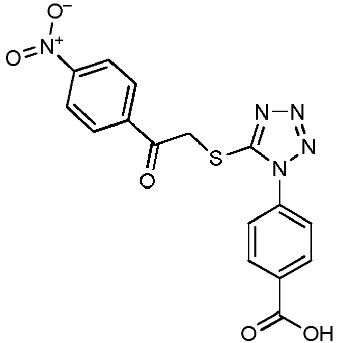

### 4-(5-((2-(4-nitrophenyl)-2-oxoethyl)thio)-1H-tetrazol-1-yl)benzoic acid

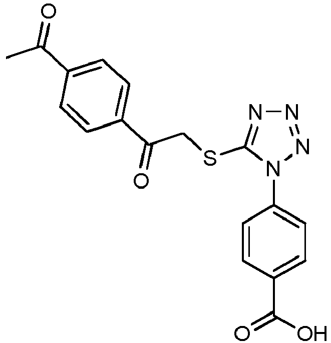

### 4-(5-((2-(4-acetylphenyl)-2-oxoethyl)thio)-1H-tetrazol-1-yl)benzoic acid

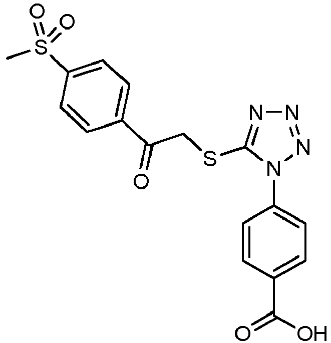

### 4-(5-((2-(4-(methylsulfonyl)phenyl)-2-oxoethyl)thio)-1H-tetrazol-1-yl)benzoic acid

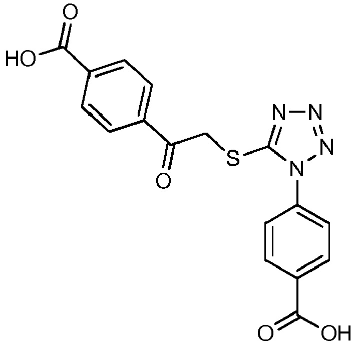

### 4-(2-((1-(4-carboxyphenyl)-1H-tetrazol-5-yl)thio)acetyl)benzoic acid

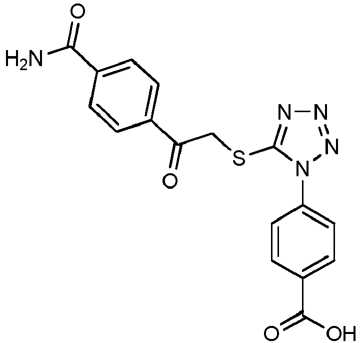

### 4-(5-((2-(4-carbamoylphenyl)-2-oxoethyl)thio)-1H-tetrazol-1-yl)benzoic acid

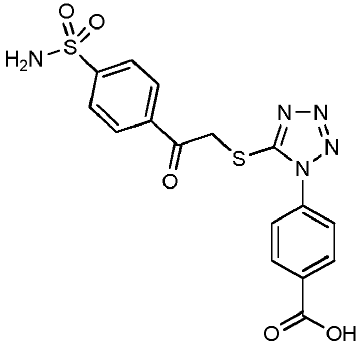

### 4-(5-((2-oxo-2-(4-sulfamoylphenyl)ethyl)thio)-1H-tetrazol-1-yl)benzoic acid

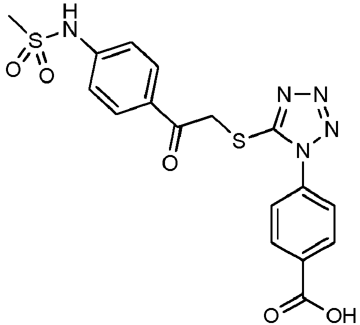

### 4-(5-((2-(4-(methylsulfonamido)phenyl)-2-oxoethyl)thio)-1H-tetrazol-1-yl)benzoic acid

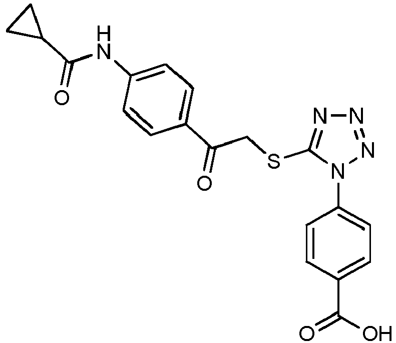

### 4-(5-((2-(4-(cyclopropanecarboxamido)phenyl)-2-oxoethyl)thio)-1H-tetrazol-1-yl)benzoic acid

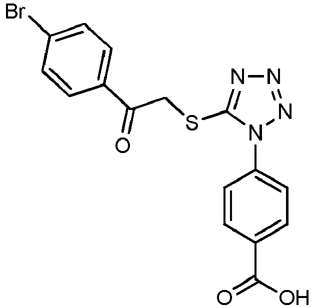

### 4-(5-((2-(4-bromophenyl)-2-oxoethyl)thio)-1H-tetrazol-1-yl)benzoic acid

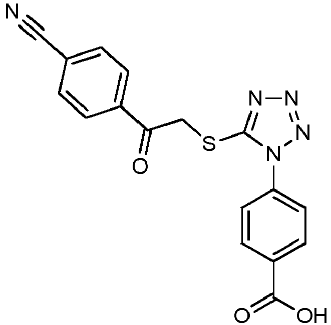

### 4-(5-((2-(4-cyanophenyl)-2-oxoethyl)thio)-1H-tetrazol-1-yl)benzoic acid

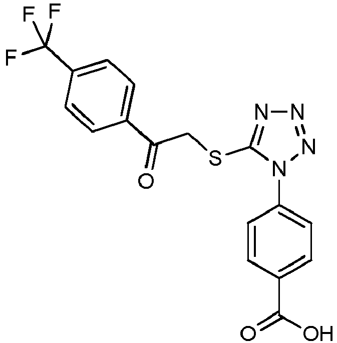

### 4-(5-((2-oxo-2-(4-(trifluoromethyl)phenyl)ethyl)thio)-1H-tetrazol-1-yl)benzoic acid

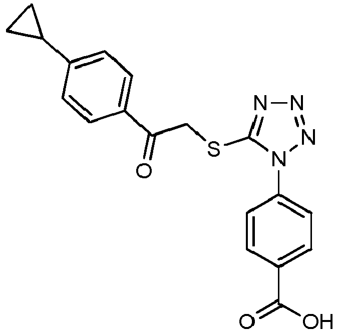

### 4-(5-((2-(4-cyclopropylphenyl)-2-oxoethyl)thio)-1H-tetrazol-1-yl)benzoic acid

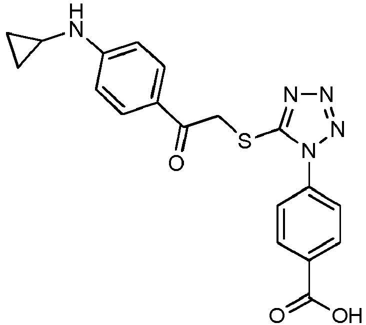

### 4-(5-((2-(4-(cyclopropylamino)phenyl)-2-oxoethyl)thio)-1H-tetrazol-1-yl)benzoic acid

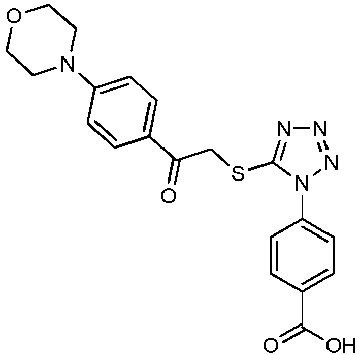

### 4-(5-((2-(4-morpholinophenyl)-2-oxoethyl)thio)-1H-tetrazol-1-yl)benzoic acid

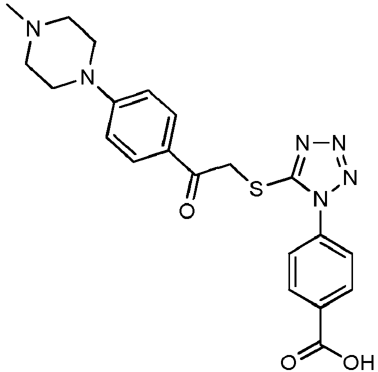

### 4-(5-((2-(4-(4-methylpiperazin-1-yl)phenyl)-2-oxoethyl)thio)-1H-tetrazol-1-yl)benzoic acid

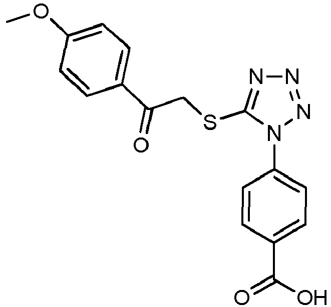

### 4-(5-((2-(4-methoxyphenyl)-2-oxoethyl)thio)-1H-tetrazol-1-yl)benzoic acid

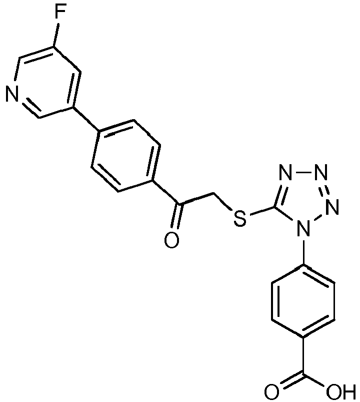

### 4-(5-((2-(4-(5-fluoropyridin-3-yl)phenyl)-2-oxoethyl)thio)-1H-tetrazol-1-yl)benzoic acid

### b) Ketoheteroaryls:

Further substructures provided by connecting the core structure to a ring structure (Cy = any cycle) are herein listed as Ketoheteroaryls. In this sense, a typical exploration in medicinal chemistry would be to replace the phenyl group present in compound #1 by a heteroaryl group which represents a similar aromatic ring with additional heteroatoms. We herein provide 6 examples of analogs that fall into this category:

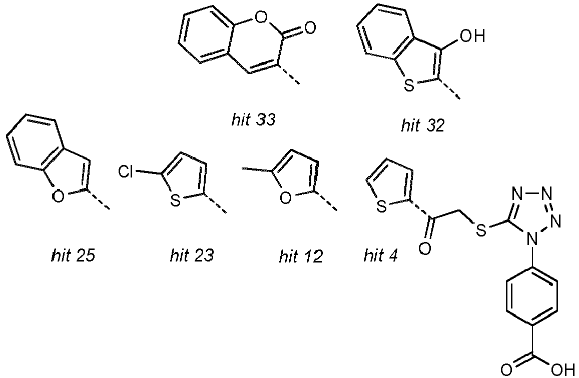

6-membered heteroaryls (like pyridines, pyrimidines, pyridazines) that are structurally closer to phenyl as illustrated below, also form part of the present invention:

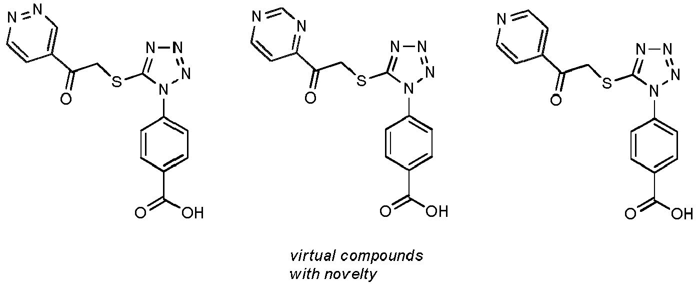

Moreover, particular examples of ketoheteroaryls compounds useful to practice the present invention are illustrated below:

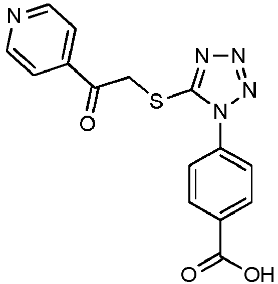

### 4-(5-((2-oxo-2-(pyridin-4-yl)ethyl)thio)-1H-tetrazol-1-yl)benzoic acid

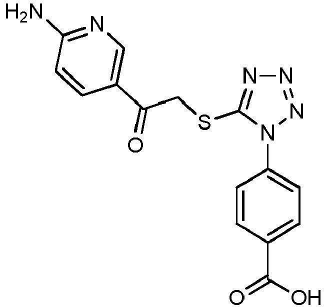

### 4-(5-((2-(6-aminopyridin-3-yl)-2-oxoethyl)thio)-1H-tetrazol-1-yl)benzoic acid

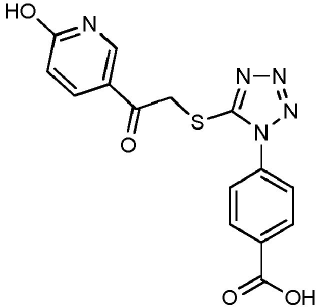

### 4-(5-((2-(6-hydroxypyridin-3-yl)-2-oxoethyl)thio)-1H-tetrazol-1-yl)benzoic acid

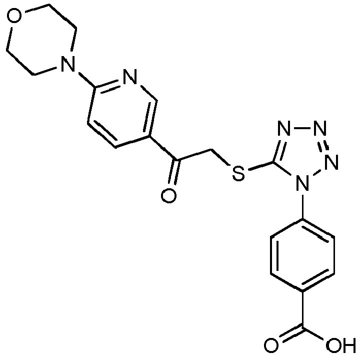

### 4-(5-((2-(6-morpholinopyridin-3-yl)-2-oxoethyl)thio)-1H-tetrazol-1-yl)benzoic acid

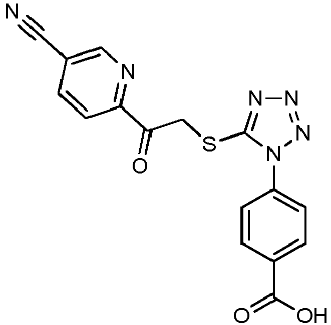

### 4-(5-((2-(5-cyanopyridin-2-yl)-2-oxoethyl)thio)-1H-tetrazol-1-yl)benzoic acid

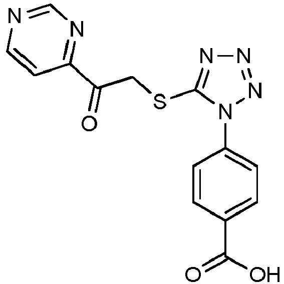

### 4-(5-((2-oxo-2-(pyrimidin-4-yl)ethyl)thio)-1H-tetrazol-1-yl)benzoic acid

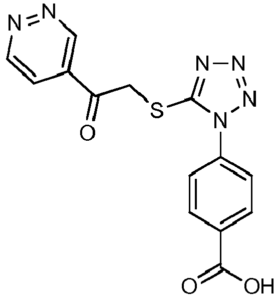

### 4-(5-((2-oxo-2-(pyridazin-4-yl)ethyl)thio)-1H-tetrazol-1-yl)benzoic acid

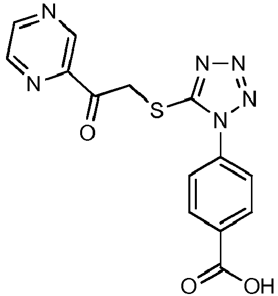

### 4-(5-((2-oxo-2-(pyrazin-2-yl)ethyl)thio)-1H-tetrazol-1-yl)benzoic acid

### c) Cyclic amides (including Compound #2):

Still further substructures provided by connecting the core structure to a ring structure (Cy = any cycle) are herein listed as Cyclic amides, as depicted below:

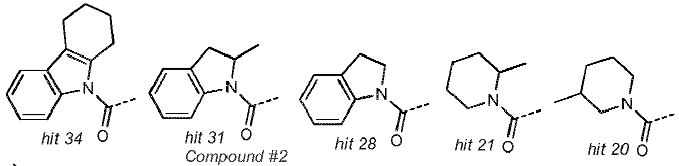

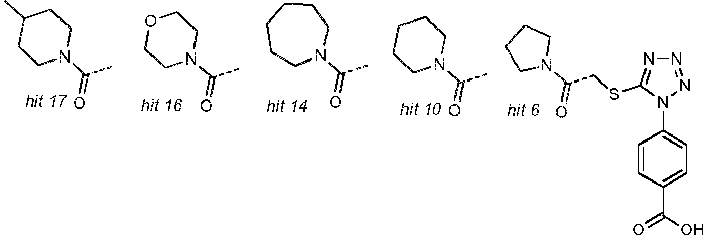

In particular, substituted indoline (and indole) analogs as illustrated below:

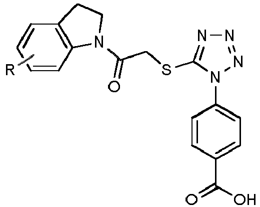

More particularly, any of the compounds substituted on the phenyl ring shown herein below:

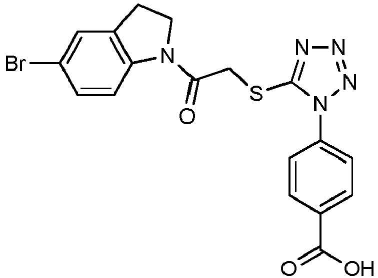

### 4-(5-((2-(5-bromoindolin-1-yl)-2-oxoethyl)thio)-1H-tetrazol-1-yl)benzoic acid

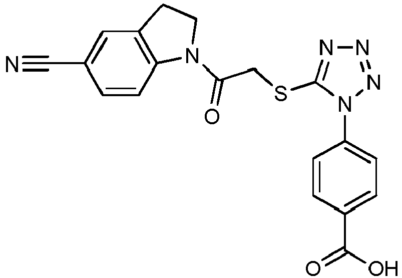

### 4-(5-((2-(5-cyanoindolin-1-yl)-2-oxoethyl)thio)-1H-tetrazol-1-yl)benzoic acid

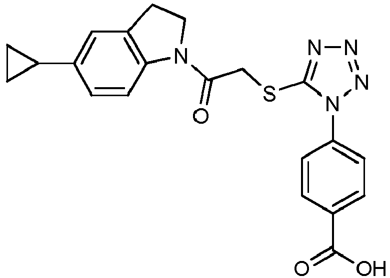

### 4-(5-((2-(5-cyclopropylindolin-1-yl)-2-oxoethyl)thio)-1H-tetrazol-1-yl)benzoic acid

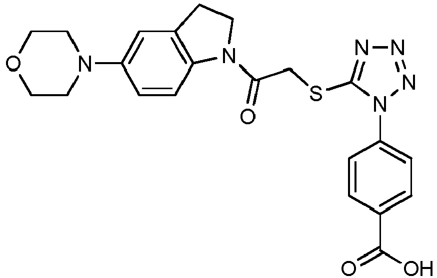

### 4-(5-((2-(5-morpholinoindolin-1-yl)-2-oxoethyl)thio)-1H-tetrazol-1-yl)benzoic acid

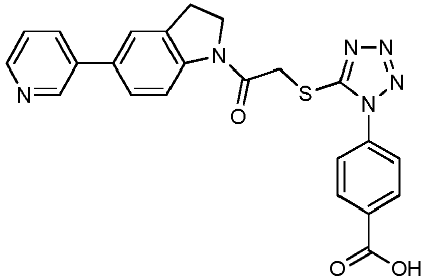

### 4-(5-((2-oxo-2-(5-(pyridin-3-yl)indolin-1-yl)ethyl)thio)-1H-tetrazol-1-yl)benzoic acid

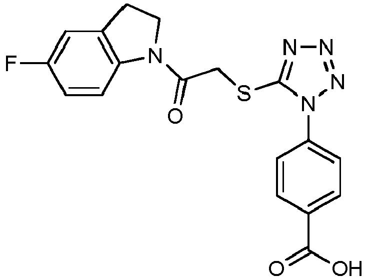

### 4-(5-((2-(5-fluoroindolin-1-yl)-2-oxoethyl)thio)-1H-tetrazol-1-yl)benzoic acid

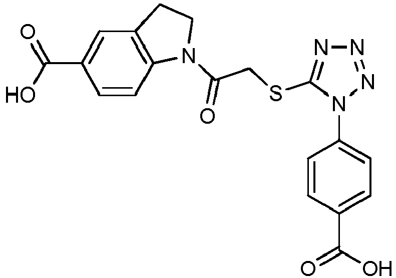

### 1-(2-((1-(4-carboxyphenyl)-1H-tetrazol-5-yl)thio)acetyl)indoline-5-carboxylic acid

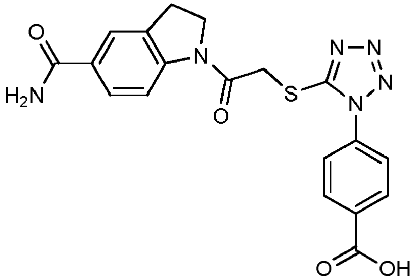

### 4-(5-((2-(5-carbamoylindolin-1-yl)-2-oxoethyl)thio)-1H-tetrazol-1-yl)benzoic acid

With modifications in other parts of the molecule:

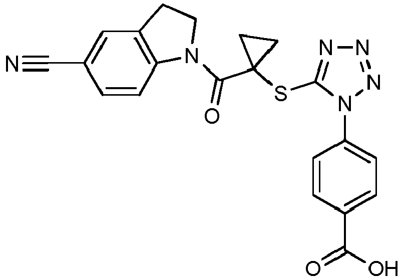

### 4-(5-((1-(5-cyanoindoline-1-carbonyl)cyclopropyl)thio)-1H-tetrazol-1-yl)benzoic acid

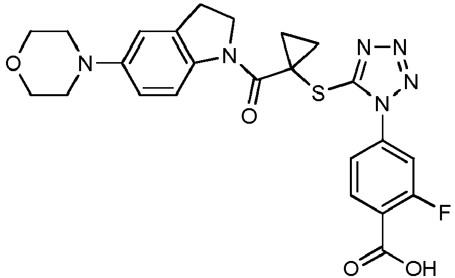

### 2-fluoro-4-(5-((1-(5-morpholinoindoline-1-carbonyl)cyclopropyl)thio)-1H-tetrazol-1-yl)benzoic acid

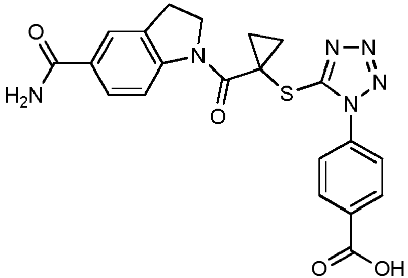

### 4-(5-((1-(5-carbamoylindoline-1-carbonyl)cyclopropyl)thio)-1H-tetrazol-1-yl)benzoic acid

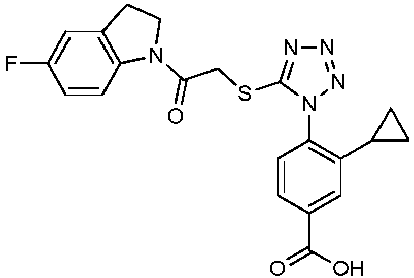

### 3-cyclopropyl-4-(5-((2-(5-fluoroindolin-1-yl)-2-oxoethyl)thio)-1H-tetrazol-1-yl)benzoic acid

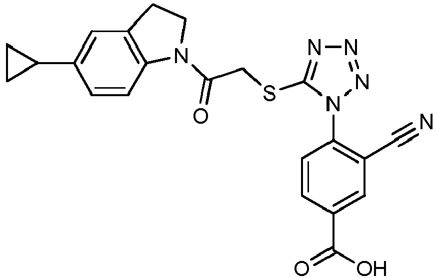

### 3-cyano-4-(5-((2-(5-cyclopropylindolin-1-yl)-2-oxoethyl)thio)-1H-tetrazol-1-yl)benzoic acid

In addition, the different substructures a) to c) above provided by connecting the core structure to a ring structure (Cy = any cycle) may be further substituted as follows:

### 1. Examples of structures having a substitution on the mercaptoacetyl linker

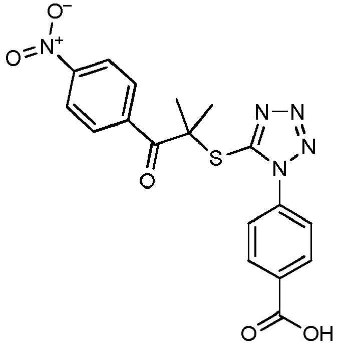

### 4-(5-((2-methyl-1-(4-nitrophenyl)-1-oxopropan-2-yl)thio)-1H-tetrazol-1-yl)benzoic acid

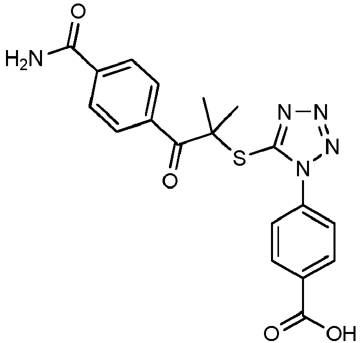

### 4-(5-((1-(4-carbamoylphenyl)-2-methyl-1-oxopropan-2-yl)thio)-1H-tetrazol-1-yl)benzoic acid

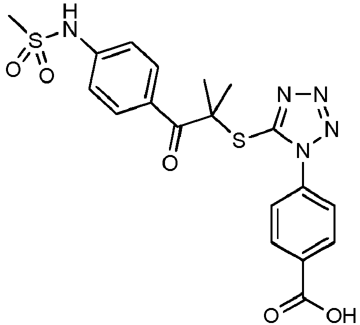

### 4-(5-((2-methyl-1-(4-(methylsulfonamido)phenyl)-1-oxopropan-2-yl)thio)-1H-tetrazol-1-yl)benzoic acid

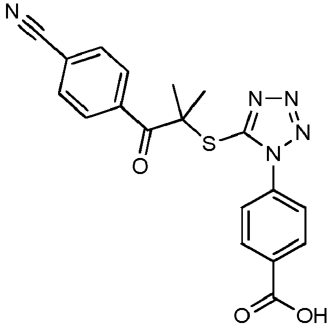

### 4-(5-((1-(4-cyanophenyl)-2-methyl-1-oxopropan-2-yl)thio)-1H-tetrazol-1-yl)benzoic acid

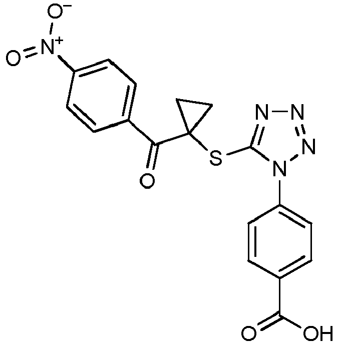

### 4-(5-((1-(4-nitrobenzoyl)cyclopropyl)thio)-1H-tetrazol-1-yl)benzoic acid

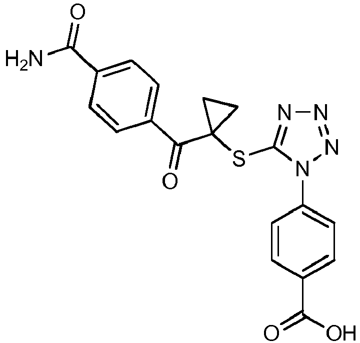

### 4-(5-((1-(4-carbamoylbenzoyl)cyclopropyl)thio)-1H-tetrazol-1-yl)benzoic acid

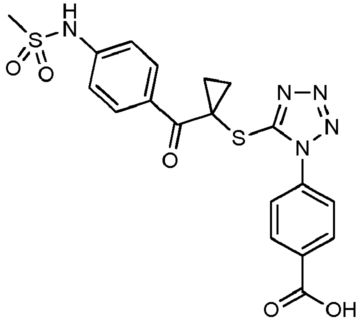

### 4-(5-((1-(4-(methylsulfonamido)benzoyl)cyclopropyl)thio)-1H-tetrazol-1-yl)benzoic acid

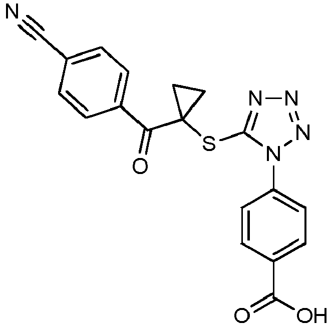

### 4-(5-((1-(4-cyanobenzoyl)cyclopropyl)thio)-1H-tetrazol-1-yl)benzoic acid

### 2. Examples of structures having a substitution on the carboxyphenyl moiety

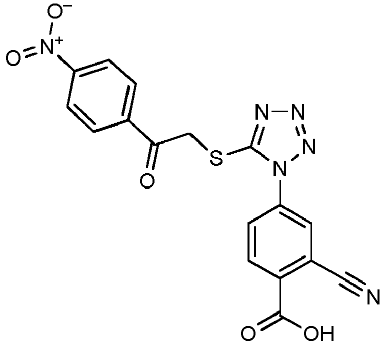

### 2-cyano-4-(5-((2-(4-nitrophenyl)-2-oxoethyl)thio)-1H-tetrazol-1-yl)benzoic acid

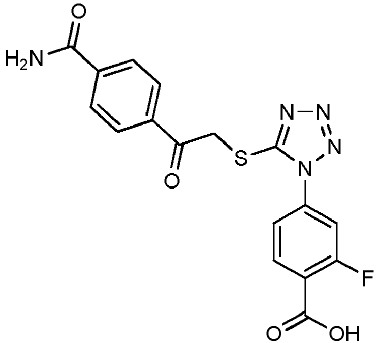

### 4-(5-((2-(4-carbamoylphenyl)-2-oxoethyl)thio)-1H-tetrazol-1-yl)-2-fluorobenzoic acid

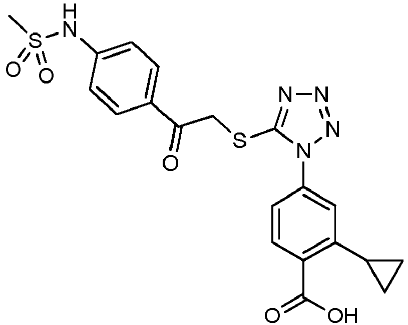

### 2-cyclopropyl-4-(5-((2-(4-(methylsulfonamido)phenyl)-2-oxoethyl)thio)-1H-tetrazol-1-yl)benzoic acid

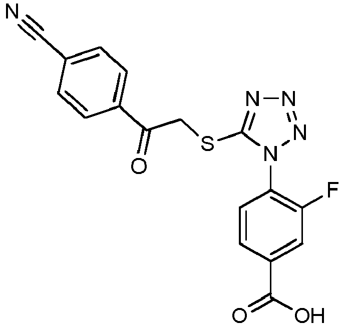

### 4-(5-((2-(4-cyanophenyl)-2-oxoethyl)thio)-1H-tetrazol-1-yl)-3-fluorobenzoic acid

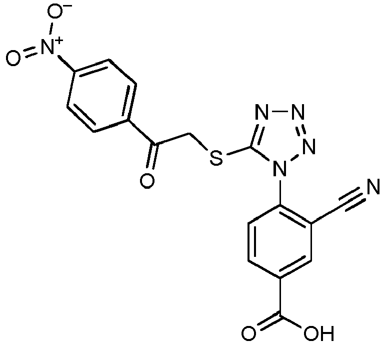

### 3-cyano-4-(5-((2-(4-nitrophenyl)-2-oxoethyl)thio)-1H-tetrazol-1-yl)benzoic acid

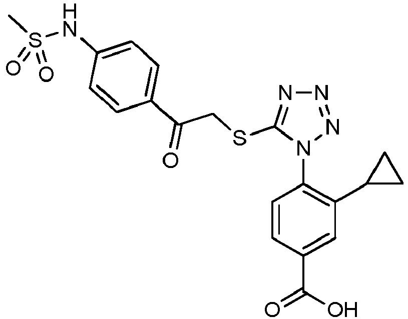

### 3-cyclopropyl-4-(5-((2-(4-(methylsulfonamido)phenyl)-2-oxoethyl)thio)-1H-tetrazol-1-yl)benzoic acid

On the other hand, an entirely new group or class of compounds that derives from extending the core structure with a nitrogen atom, a carbon atom and a cyclic group to provide compounds resembling compound #6, is herein provided under the subclass benzylamides:

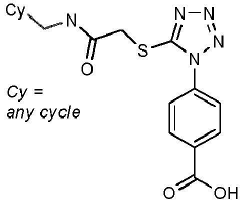

Compounds falling within this category are herein indicated below:

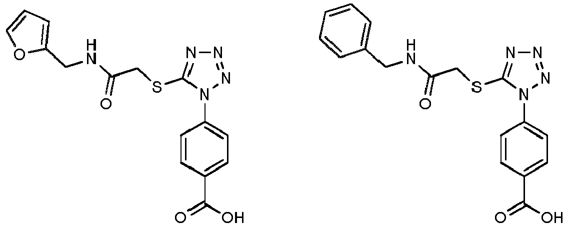

Other compounds pertaining to this class of compounds are:

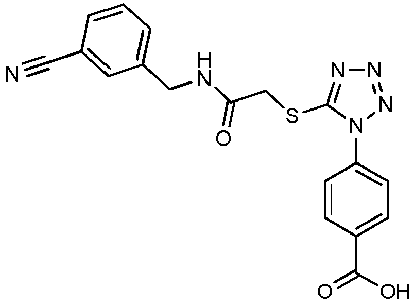

### 4-(5-((2-((3-cyanobenzyl)amino)-2-oxoethyl)thio)-1H-tetrazol-1-yl)benzoic acid

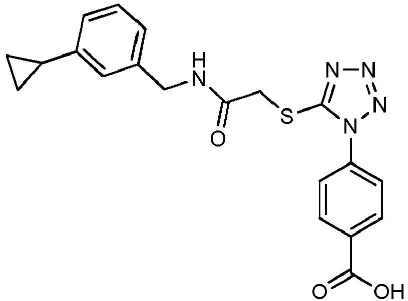

### 4-(5-((2-((3-cyclopropylbenzyl)amino)-2-oxoethyl)thio)-1H-tetrazol-1-yl)benzoic acid

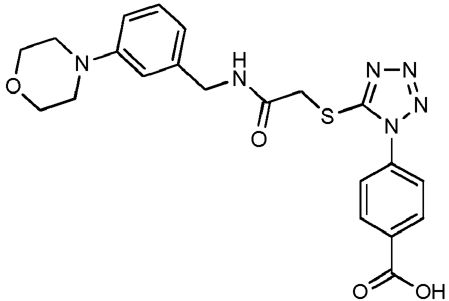

### 4-(5-((2-((3-morpholinobenzyl)amino)-2-oxoethyl)thio)-1H-tetrazol-1-yl)benzoic acid

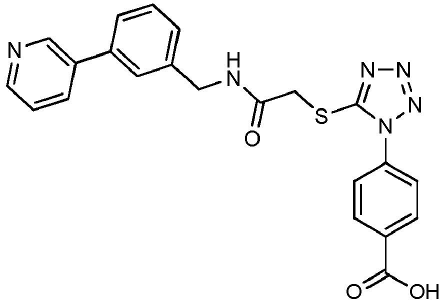

### 4-(5-((2-oxo-2-((3-(pyridin-3-yl)benzyl)amino)ethyl)thio)-1H-tetrazol-1-yl)benzoic acid

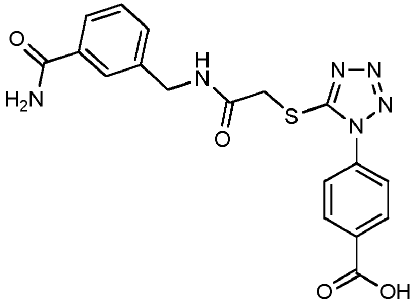

### 4-(5-((2-((3-carbamoylbenzyl)amino)-2-oxoethyl)thio)-1H-tetrazol-1-yl)benzoic acid

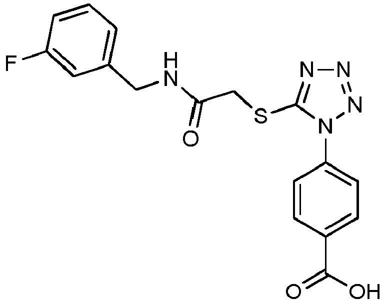

### 4-(5-((2-((3-fluorobenzyl)amino)-2-oxoethyl)thio)-1H-tetrazol-1-yl)benzoic acid

With modifications in other parts of the molecule:

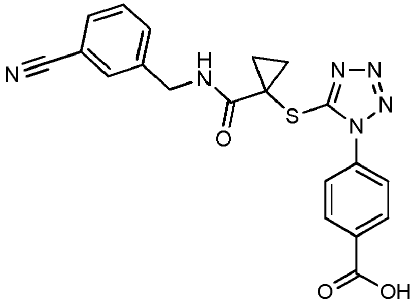

### 4-(5-((1-((3-cyanobenzyl)carbamoyl)cyclopropyl)thio)-1H-tetrazol-1-yl)benzoic acid

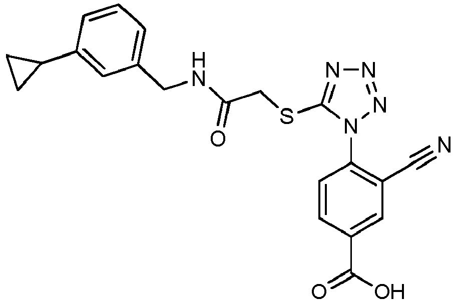

### 3-cyano-4-(5-((2-((3-cyclopropylbenzyl)amino)-2-oxoethyl)thio)-1H-tetrazol-1-yl)benzoic acid

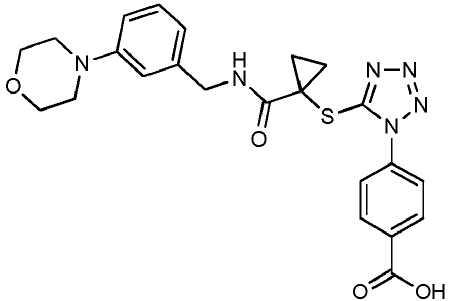

### 4-(5-((1-((3-morpholinobenzyl)carbamoyl)cyclopropyl)thio)-1H-tetrazol-1-yl)benzoic acid

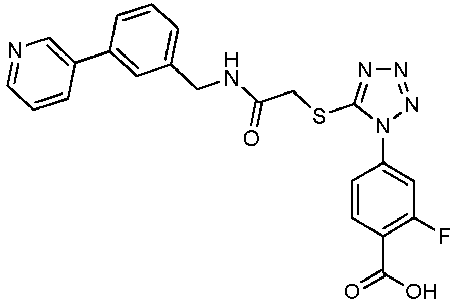

### 2-fluoro-4-(5-((2-oxo-2-((3-(pyridin-3-yl)benzyl)amino)ethyl)thio)-1H-tetrazol-1-yl)benzoic acid

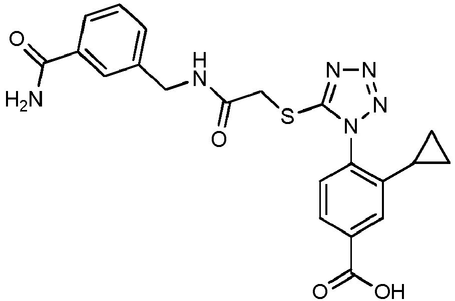

### 4-(5-((2-((3-carbamoylbenzyl)amino)-2-oxoethyl)thio)-1H-tetrazol-1-yl)-3-cyclopropyl benzoic acid

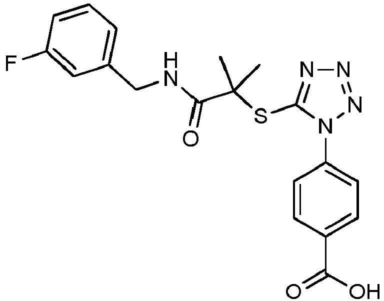

### 4-(5-((1-((3-fluorobenzyl)amino)-2-methyl-1-oxopropan-2-yl)thio)-1H-tetrazol-1-yl)benzoic acid

With heteroaryls:

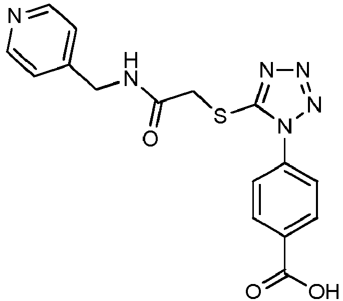

### 4-(5-((2-oxo-2-((pyridin-4-ylmethyl)amino)ethyl)thio)-1H-tetrazol-1-yl)benzoic acid

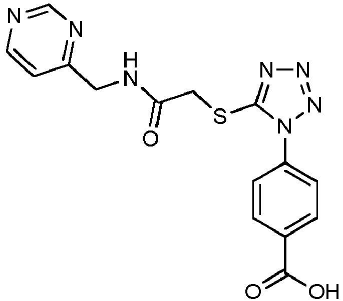

### 4-(5-((2-oxo-2-((pyrimidin-4-ylmethyl)amino)ethyl)thio)-1H-tetrazol-1-yl)benzoic acid

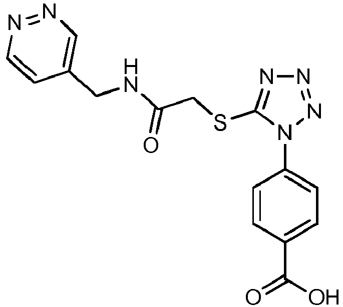

### 4-(5-((2-oxo-2-((pyridazin-4-ylmethyl)amino)ethyl)thio)-1H-tetrazol-1-yl)benzoic acid

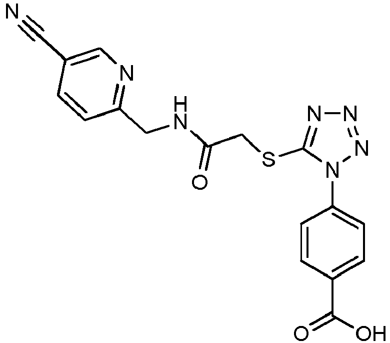

### 4-(5-((2-(((5-cyanopyridin-2-yl)methyl)amino)-2-oxoethyl)thio)-1H-tetrazol-1-yl)benzoic acid

All of the above compounds, #1, #2 and #6, as well as those grouped as structures provided by connecting the core structure to a ring structure (Cy = any cycle) to provide compounds resembling compound #1 or #2, or as a class of compounds that derives from extending the core structure with a nitrogen atom, a carbon atom and a cyclic group to provide compounds resembling compound #6, are herein named "compounds of the invention".

It is herein noted that all compounds of the invention are encompass by the following general formula: 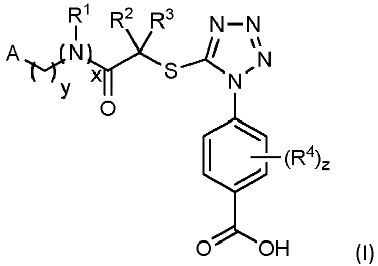 wherein:
- A represents a group selected from aryl, heteroaryl and cyclic amides optionally substituted by 1 or 2 groups that are independently selected from:
   e) halogen atom, -CN, -N(R^{a})R^{b}, -OR^{a}, -C(=O)R^{a}, -C(=O)OR^{a}, -C(=O)N(R^{a})R^{b}, -OC(=O)-R^{a}, -N(R^{c})C(=O)R^{b}, -NR^{c}SO₂R^{a}, -SO₂N(R^{a})R^{b}, -SR^{a}, -S(O)R^{a}, -S(O)₂R^{a};
   f) linear or branched C₁-C₆ alkyl, optionally substituted by 1, 2 or 3 halogen atoms;
   g) C₃-C₆ cycloalkyl which optionally contains 1 or 2 heteroatoms selected from O, S and N, and which ring is optionally substituted by C₁-C₃ alkyl;
   h) phenyl or C₅-C₆ heteroaryl each optionally substituted by halogen atom, cyano group, C₁-C₃ alkyl or cyclopropyl;
- each R^{a}, R^{b} and R^{c} independently represents:
   e) hydrogen atom,
   f) linear or branched C₁-C₁₂ alkyl, C₃-C₆ cycloalkyl and C₄-C₆ heterocycloalkyl, which are optionally substituted by 1, 2 or 3 substituents selected from a carbonyl group, halogen atom, hydroxy, phenyl, C₃-C₆ cycloalkyl, linear or branched C₁-C₆ alkoxy, amino, alkylamino, dialkylamino, linear or branched C₁-C₆ alkylcarbonyl,
   g) phenyl or C₅-C₆ heteroaryl group, which are optionally substituted by 1, 2 or 3 substituents selected from halogen atom, cyano group, linear or branched C₁-C₆ alkyl, linear or branched C₁-C₆ haloalkyl, hydroxy, linear or branched C1-C6 alkoxy, amino, alkylamino, dialkylamino;
   h) R^{a} and R^{b} form together with the nitrogen atom to which they are attached, a 3- to 8 membered ring which optionally contains a further heteroatom selected from O, N and S, and which ring is optionally substituted by 1, 2 or 3 substituents selected from carbonyl group, linear or branched C₁-C₆ alkyl, linear or branched C₁-C₆ haloalkyl, linear or branched C₁-C₆ alkylcarbonyl;
- x and y are integers independently selected from 0 and 1;
- R¹ represents a group selected from hydrogen, cyclopropyl or linear or branched C₁-C₆ alkyl, wherein said alkyl is optionally substituted by 1, 2 or 3 halogen atoms;
- when y is 0, then 2 or 3 carbon atoms of R¹ can form a 5- or 6-membered ring together with the neighboring nitrogen atom and the 2 adjacent carbon atoms of the aromatic ring to which the nitrogen is attached;
- each R² and R³ independently represent a group selected from hydrogen, cyclopropyl or linear or branched C₁-C₆ alkyl; optionally R² and R³ can form a 3-or 4-membered spiro ring together with the carbon atom to which they are both attached;
- z is an integer selected from 0, 1, 2 or 3;
- R⁴ represents a group selected from -CN, cyclopropyl or linear or branched C₁-C₆ alkyl, said alkyl is optionally substituted by 1, 2 or 3 halogen atoms; wherein the group R⁴, if present, replaces the hydrogen atom of one of the groups CH present in the phenyl ring to which R⁴ is attached;
as well as any pharmaceutically acceptable salts thereof.

Preferably, each R2 and R3 independently represents a group selected from cyclopropyl or linear or branched C1-C6 alkyl.

More preferably, R2 and R3 form a 3-or 4-membered spiro ring together with the carbon atom to which they are both attached.

More preferably, z is an integer selected from 1, 2 or 3; R4 represents a group selected from-CN, cyclopropyl or linear or branched C1-C6 alkyl, wherein said alkyl is optionally substituted by 1, 2 or 3 halogen atoms; and wherein the group R4 replaces the hydrogen atom of one of the groups CH present in the phenyl ring to which R4 is attached.

More preferably, one or both of the integers x and y equal 1 and A represents a group selected the list consisting of aryl, heteroaryl and cyclic amides substituted by 1 or 2 groups that are independently selected from halogen atom, -CN, -N(Ra)Rb, -ORa, -C(=O)Ra, -C(=O)ORa, -C(=O)N(Ra)Rb, -OC(=O)-Ra, -N(Rc)C(=O)Rb, -NRcSO2Ra, -SO2N(Ra)Rb, -SRa, -S(O)Ra, -S(O)2Ra, linear or branched C1-C6 alkyl, wherein said alkyl is optionally substituted by 1, 2 or 3 halogen atoms; C3-C6 cycloalkyl which optionally contains 1 or 2 heteroatoms selected from O, S and N, and which ring is optionally substituted by C1-C3 alkyl; phenyl or C5-C6 heteroaryl each optionally substituted by halogen atom, C1-C3 alkyl or cyclopropyl.

Still more preferably, the compounds of the invention are any of compounds #1, #2 or #6, or any of those identified above and grouped as structures provided by connecting the core structure to a ring structure (Cy = any cycle) to provide compounds resembling compound #1 or #2, or any of those identified above and grouped as a class of compounds that derives from extending the core structure with a nitrogen atom, a carbon atom and a cyclic group to provide compounds resembling compound #6.

The compounds of the present invention can be in a free form or in the form of a pharmaceutically acceptable salt.

Examples of pharmaceutically acceptable salts include inorganic acid salts such as hydrochloride, sulfate, nitrate, phosphate or hydrobromide, etc., organic acid salt such as acetate, fumarate, oxalate, citrate, methanesulfonate, benzenesulfonate, p-toluenesulfonate or maleate, etc. Also, when the compound has a substituent such as carboxyl group, there may be mentioned a salt with a base (for example, alkali metal salt such as sodium salt, potassium salt, etc. or alkaline earth metal salt such as calcium salt, etc.).

The compounds of the present invention or their enantiomers or pharmaceutically acceptable salts can be in any of its intramolecular salt or adduct, or its solvate or hydrate.

When the compounds of the present invention or a pharmaceutically acceptable salt thereof of the present invention is used as an effective ingredient for medical use, it can be used with a pharmaceutically acceptable carrier. A pharmaceutically acceptable carrier is an inert carrier suitable for each administration method, and can be formulated into conventional pharmaceutical preparation (tablets, granules, capsules, powder, solution, suspension, emulsion, injection, infusion, etc.). As such a carrier, there may be mentioned, for example, a binder (such as gum arabic, gelatin, sorbitol and polyvinylpyrrolidone), an excipient (such as lactose, sugar, corn starch and sorbitol), a lubricant (such as magnesium stearate, talc and polyethylene glycol), a disintegrator (such as potato starch) and the like, which are pharmaceutically acceptable. When they are used as an injection solution or an infusion solution, they can be formulated by using distilled water for injection, physiological saline, an aqueous glucose solution.

The administration method of the compounds of the present invention and/or a pharmaceutically acceptable salts thereof of the present invention is not particularly limited, and a usual oral or parenteral administration method (intravenous, intramuscular, subcutaneous, percutaneous, intranasal, and as others, transmucosal, enteral, etc.) can be applied.

The dosage of the compounds of the present invention or a pharmaceutically acceptable salts thereof of the present invention may be optionally set in a range of an effective amount sufficient for showing a pharmacological effect, in accordance with the potency or characteristics of the compound to be used as an effective ingredient. The dosage may vary depending on administration method, age, body weight or conditions of a patient.

The following examples are merely illustrative of the present invention and do not limit the same.

### Examples

### Example 1. SYNTHESIS

The compounds listed through-out the present specification can be prepared following the general synthetic route below:

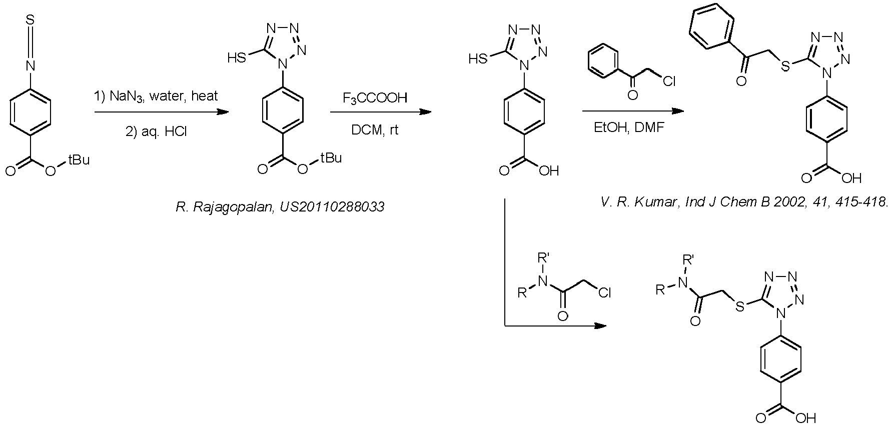

### Example 2.

### 2.1. MATERIALS AND METHODS

**Protein and ligands models.** The X-ray crystal structure of the phosphotyrosine-binding domain of Hakai (PDB 3VK6) was downloaded from the Protein Data Bank and the dimer modelled using the proper symmetry operations. Amino acid protonation was carried out using the pdb2pqr server at a pH of 7.2. 3D models for the ligands were built using the Virtual Screening and Data Management Integrated Platform (VSDMIP), as described elsewhere. Briefly, the initial 3D coordinates for each ligand were generated with CORINA [Sadowski, J.; Gasteiger, J.; Klebe, G. Comparison of Automatic Three-Dimensional Model Builders Using 639 X-Ray Structures. J. Chem. Inf. Comput. Sci. 1994, 34, 1000-1008 (DOI: 10.1021/ci00020a039)]. Thereafter, ALFA [4] was used to generate a large variety of conformers for each of which MOPAC-calculated atomic partial charges were assigned by employing the AM1 semiempirical model and the ESP method. All ligand models were stored in the VSDMIP database to be used in the different virtual screening campaigns.

**Virtual Screening.** Ligands in the eMolecules catalogue [https://www.emolecules.com/info/products-screening-compounds.html] were downloaded and processed as described in the preceding section. Only molecules presenting a carboxylic acid moiety and/or a phosphate group capable of mimicking a phosphotyrosine residue were considered. Next, CRDOCK was used to lodge the selected molecules inside the binding pocket of Hakai by using the CRScore scoring function and the BFGS energy minimizer. The ligands were then ranked according to the predicted score and the top 350 molecules were reevaluated by using an in-house implementation of the HYDE scoring function. Finally, the best 20 molecules were visually inspected to select a final set of 6 molecules.

**Binding pocket analysis.** To better analyse the results of the virtual screening campaign, we used our in-house cGRILL software [6] to produce affinity maps within the binding pocket of Hakai's phosphotyrosine-binding domain based on the van der Waals, Coulombic and hydrogen bonding interactions of hypothetical atomic probes. The negatively charged acceptor probe (=O) was used to map possible locations for the molecular recognition of the phosphotyrosine residue to help filtering the docking solutions during the visual inspection of the poses.

### Plasmids, inhibitors and antibodies

pcDNA-Flag-Hakai, pBSSR-HA-ubiquitin, pSG-v-Src and pcDNA-myc-E-Cadherin plasmids were previously described. Compounds Hakin-1 [4-(5-{[2-(4-nitrophenyl)-2-oxoethyl]thio}-1H-tetrazol-1-yl)benzoic acid] and Hakin-5 [(2E,4E,8E)-7,13-Dihydroxy-4,8,12-trimethyl-2,4,8-tetradecatrienoic acid] were obtained from ChemBridge Corporation and TimTec or Analyticon Discovery, respectively. The rest of the analogues tested (Ketophenyls A-1, A-7, A-8, A-9; ketoheteroaryls: A-23, A-25; Cyclic amides: A- 10, A-16 and Bencylamide: A-6.1) were obtained from Vitasmlab. Compounds were re-suspended in DMSO (Sigma) at 100mM for *in vitro* assays, and Hakin-1 was at 100mM for *in vivo* assays. The highest concentration of DMSO was used as the vehicle control for the experiments. Note that Hakin-5 chemical structure is in figure 1.

### Cell culture

MDCK, HEK293T, HepG2, MCF7 and ACHN cells were cultured in Dulbecco's Modified Eagles Medium (DMEM). MDCK stably expressing Hakai cells (Hakai-MDCK) were previously reported and were growth in DMEM with G418 (800 µg/ml). Different clones of Hakai-MDCK cells shown comparable phenotypes and characteristics as demonstrated previously. LoVo and PC-3 cells were cultured in F-12K Medium (Kaighn's Modification of Ham's F-12 Medium) and HT-29 cells in McCoy's 5a Medium Modified. 5637 cells were cultured in RPMI medium. All culture media were supplemented with 1% penicillin/streptomycin and 10% of heat-inactivated fetal bovine serum (FBS) at 37ºC in a humidified incubator with 5% CO2. Cells were monthly tested for mycoplasma contamination and used only for 1-3 months after defrosted. LoVo and HT29 cells were authenticated with the StemElite ID system (Promega). For phase-contrast images, culture cells were photographed with a Nikon Eclipse-TI microscope.

### Ubiquitination assays

For ubiquitination assays, 750.000 HEK293T cells were seeded in 6-well cell culture plates and after 24h were transfected with 0.25 µg Src, 0.75 µg Flag-Hakai, and 0.5 µg HA-ubiquitin with Lipofectamin 2000 (Invitrogen, UK). Six hours after transfection cells were treated with indicated concentrations of Hakin-1, Hakin-5 or the rest of the analogues tested for 36h. Whole cell extracts were obtained in lysis buffer (20 mM Tris-HCI pH 7.5, 150 mM NaCl and 1% Triton X-100) containing 10 µg/ml leupeptin, 10 µg/ml aprotinin and 1 mM phenylmethanesulphonyl fluoride (PMSF), supplemented with 10 mM N-ethylmaleimide. Cells were harvested and subjected to western blotting using anti-HA antibody to detect ubiquitination.

### Immunoprecipitation

For immunoprecipitation experiments, 293 cells were transfected with 3 µg Src, 4 µg Flag-Hakai, and 2 µg HA-ubiquitin and 3 µg E-cadherin with Lipofectamin 2000 (Invitrogen, UK). 24 h after transfection, cells were lysed for 20 min in 1 ml of 1% Triton X-100 lysis buffer (20 mM Tris-HCI pH 7.5, 150 mM NaCl and 1% Triton X-100) containing 10 µg/ml leupeptin, 10 µg/ml aprotinin and 1 mM phenylmethanesulphonyl fluoride (PMSF), supplemented with 10 mM N-ethylmaleimide and 2.5 mM sodium orthovanadate. After centrifugation at 18.000g for 10 min, the supernatants were immunoprecipitated for 2 h with 2 µg of anti-E-cadherin antibody bound to 60 µl of protein G PLUS-Agarose beads, followed by SDS-polyacrylamide gel electrophoresis (PAGE) and western blotting with the indicated antibodies as previously reported.

### Viability assays

For cytotoxicity assays, 1 x 10⁴ cells were seeded per well into a 96-well plate. After 24h cells were treated with the indicated inhibitors for 72h and a MTT colorimetric cell viability assay was performed following manufacturer's instructions (Sigma Aldrich, St Louis,MO). Absorbance was measured at 570 and 630nm using a Multiskan Plus Reader (Nanoquant Infinite M200 Tecan Trading AG, Switzerland). Dose-response curves were designed with GraphPad Prism Software and the half-maximal inhibitory concentration (IC₅₀) values were calculated. Represented data are the mean ± SEM of at least three independent experiments with six replicates per condition.

### Western blotting and immunofuorescence

For western blot analysis, cells were treated with the indicated inhibitors for 48h and the whole cell extracts were obtained as described previously. Twenty micrograms of lysates were resolved on a 10% polyacrilamide SDS-PAGE followed by western blot analysis performed as previously described. For immunofluorescence assays cells were grown for 24h on glass coverslips and treated with the indicated inhibitors for 48h. Cells were fixed with 4% PFA for 15 min, permeabilized with 0.5% Triton X-10 and incubated with E-cadherin antibody for 2h. Coverslips were incubated with fluorescein-tagged secondary antibody (Dakopatts, Sweden) for 1. Finally coverslips were mounted with ProLong Gold antifade reagent (LifeTech, UK) and images were taken in epifluorescence microscope (Olympus) using 40X objective.

### Proliferation assays

For BrdU assays, 1x10⁴ of indicated cells were plated per well into a 96-well plate. After 24 h, cells were treated with the indicated inhibitors for 48 h. Three independent experiments were plated with six replicates per each condition. Cells were treated with 10 mM BrdU for 2 h. BrdU incorporation into newly synthesized DNA was measured using a cell proliferation colorimetric immunoassay kit according to the manufacturer's instructions (Roche, Switzerland). Results are expressed as mean ± S.D. Results are represented as percentage of positive cells (mean ± S.D) of three independent experiments.

### Soft agar-colony formation assay

Soft agar-colony formation assay was performed on 12-well plates in triplicates at a density of 5x10³ MDCK and MDCK-Hakai cells/well, or 12x10³ HT29 cells/well. Cells were seeded in medium with 0.5% low-melting agarose over a layer with 0.75% low-melting agarose (Lonza Rockland,, ME, USA). Cells were treated with the indicated inhibitors and DMSO was used as vehicle. Treatment was refreshed every 3 days and, after 21 days for MDCK and MDCK-Hakai cells or 28 days for HT29 cells, number of colonies were quantified. Quantification of five randomly-selected fields of each condition was photographed with a Nikon Eclipse-TI microscope (objective 4x). Experiments were conducted with three triplicates and were repeated three times. Data are represented as mean ± SD.

### Migration and Invasion assay

For invasion assays, cells were treated with Hakin-1 or DMSO as vehicle for 48h using 1% FBS during the last 24 hours. 3x10⁵ MDCK, MDCK-Hakai or LoVo cells were seeded in a cell invasion chamber (Cell invasion assaykit, Chemicon International) containing medium with 2% FBS. After 72 hours for MDCK and MDCK-Hakai Invasive and 16h for LoVo cells, invasive cells invaded that reach the lower chamber containing 30% FBS were fixed and stained with crystal violet (Sigma Aldrich, St Louis, MO) following the manufacturer's specifications. For migration assays, HT29 cells were cultured with Hakin-1 or DMSO as vehicle for 48h, using medium without serum the last 24h. In the cell migration chamber were seeded 3x10⁵ HT29 cells (Cell migration kit, Millipore, Bedford, MA) containing medium without serum. After 16 h, migrated cells in the lower chamber containing serum with 30% FBS were stained with crystal violet and counted following the manufacturer's specifications. For both invasion and migration assays cells were counted in five fields photographed with an Olympus microscope using a 20x objective, experiments were performed in triplicates for each condition and the assays were repeated at least three times. Results are expressed as mean ± SD.

### Tumour xenograft model

Xenografts experiments were performed in Experimental Surgery Unit - Technological Training Center from INIBIC in compliance with the European Community Law (86/609/EEC) and the Spanish law (R.D. 53/2013). The experiment was approved by the Ethics Committee for Animal Experimentation of Xerencia de Xestion Integrada da Coruña (XXIAC). Mice were in a 12/12 hours light/dark cycle with water and food available ad libitum. Six weeks old athymic nu/nu mice were randomly distributed in groups. One million of MDCK cells, resuspended in DMEM without serum and antibiotic, were subcutaneously inoculated in both flanks in two groups of 3 animals. The same number of Hakai-MDCK cells were injected in two groups of 4 animals. Twenty days after inoculation tumours in Hakai-MDCK were palpable. Then, half of the animals were treated with Hakin-1 (5mg/kg) and the other half with the same concentration of DMSO every 3 days. Tumour outgrowth was monitored twice a week taking measurements of tumour length (L) and width (W) with an electronic calipter. Tumour volume was calculated as pLW2/6. Forty days after inoculation, animals were sacrificed. Tumours, lungs, kidneys and livers were collected and fixed in 4% PFA and embedded in paraffin blocks for histology and/or immunohistochemistry (IHC) analyses.

### Histology and Immunohistochemistry

Tumours and tissues were deparaffinised, rehydrated and stained with haematoxylin and eosin (H&E) as previously described. Tumour sections (4 µm) were also deparaffinised and hydrated for immunohistochemistry. Antigen retrieval was carried by heating the samples (2100 Retriever; PickCell Laboratories) in citrate buffer (Dako REAL, Denmark) or in EDTA buffer. Then, endogenous peroxidase activity was blocked with peroxidase blocking (DakoCytomation, Denmark). Samples were blocked and permeabilized with 0.2 %BSA and 0.1% Tx-100 for 1 hour and incubated with the indicated primary antibodies overnight at 4ºC in a wet chamber. Slides were incubated for 1 hour at room temperature with the secondary antibody and detection was carried our using DAB (DakoReal Envision kit) according to manufacturer instructions. Finally, nuclei were counterstained with Gill's Hematoxylin and mounted with DePeX. Pictures were taken with an Olympus microscope. Quantification of images was performed taken 5 photographs of each animal with Image J programme and the represented results are shown as mean ± SEM. The number of mitosis was counted in sections stained with H&E. In this case, ten pictures of each tumour were taken with an Olympus BX50 microscope (objective 40x) and the number of mitosis was counted manually. Results are represented as mean ± SEM and a representative photograph is shown for each condition.

### Quantification of lung metastasis from in vivo mouse model

Real-time PCR was used to study the presence of metastasis in the lung mice. Primers for HA epitope and Hakai present in ectopic HA-tagged Hakai expressed in MDCK-Hakai cells (5'-TCTGGGACGTCGTATGGGTA-3'; 5'-TTCTTCATCACCTTGCGGG-3') were used for the quantification. Primers for mouse apolipoprotein B (*apob*) (5'- CGTGGGCTCCAGCATTCTA-3'; 5'- TCACCAGTCATTTCTGCCTTTG-3') were used as endogenous control. MDCK cell line was used as negative control. Lung DNA was extracted from 10-15 sections of paraffin blocks (4 µm) using with QIAamp DNA Mini Kit (Qiagen). The amplification and quantification of DNA was carried by quantitative PCR in technical triplicates by using a LightCycler 480 real-time lightcycler (Roche). Relative DNA levels were calculated by 2^{-ΔΔCt} method.

### Statistical analysis

Shapiro-Wilk test was used to check a normal distribution and Levene test to assess the equality of variances. Statistical significance of data was determined with ANOVA with Bonferroni test or Kruskal-Wallis with Tukey correction test. Significance among the experimental groups indicated in the figures is shown as * p < 0.05, **p < 0.01 and ***p < 0.001. Results obtained are expressed as mean ± SD or mean ± SEM as indicated. Survival graphic in xenograft assay was analysed with GraphPad Prism software and the test of Breslow was used to calculate *p* values. Results are represented as fold induction of treated cells over the values obtained in the untreated cells.

**List of antibodies used to carry out the present invention.**

| **Antibody** | **Dilution** | **Use** | **Catalog n° / provider** |
|---|---|---|---|
| **Hakai** | 1:1000 | Western | Invitrogen 36-2800 |
| | 1:250 | IHQ(P) | Invitrogen 36-2800 |
| **E-Cadherin** | 1:1000 | Western | BD Trans Lab 610182 |
| | 2 ug | IP | BD Trans Lab 610182 |
| | 1:200 | IF | BD Trans Lab 610182 |
| | 1:400 | IHQ(P) | Cell signaling 243E10 |
| **Cortactin** | 1:1000 | Western | Millipore 05-180 |
| | 1:50 | IHQ(P) | Millipore 05-180 |
| **N-Cadherin** | 1:1000 | Western | Abcam ab18203 |
| | 1:100 | IHQ(P) | Abcam ab18203 |
| **Vimentin** | 1:1000 | Western | Cell signalling D21H3 |
| **Ki67** | 1:150 | IHQ(P) | Dako M7240 |
| **CD31** | 1:100 | IHQ(P) | Abcam ab 28364 |
| **HA** | 1:1000 | Western | Roche 12CA5 |
| **FLAG** | 1:4000 | Western | Sigma Aldrich, Clone M (F1316S) |
| **GAPDH** | 1:5000 | Western | Invitrogen 39-8600 |
| **HRP rabbit** | 1:5000 | Western | GE healthcare NA934 |
| **HRP mouse** | 1:5000 | Western | GE healthcare NA931 |
| **Mouse IgG** | 2 µg | IP | Santa Cruz Biotechnology sc-2025 |

### In vivo TUNEL assay

Tissue sections from tumours were deparaffinised and rehydrated using standard protocols. The slides were rinsed twice with PBS and treated with citrate buffer buffer (Dako REAL, Denmark) in microwave at 350W for 5 min. The tissue sections were then analysed with an *in situ* Cell Death Detection Kit, Fluorescein (Roche) following the manufacturer's instructions. Then, slides were incubated with Hoechst for 5 min in darkness. The reaction was visualized under an epifluorescence Olympus microscope using 20x objective. Five representative pictures of each section were taken. The percentage of positive cells was calculated and results are represented as mean ± SEM.

### 2.2. RESULTS

### Identification of putative selective Hakai inhibitors

With the aim of finding candidate molecules with the required potential to inhibit Hakai, we designed a virtual screening workflow based on the structural information available and the nature of the phosphotyrosine-binding pocket, which was explored with the aid of affinity probes. As a first step, we considered only molecules in our chemical library that display a negatively charged carboxylate or phosphate group that would be complementary to the highly positive molecular electrostatic potential of the binding pocket. The selected molecules were then docked into the Hakai dimer to evaluate all possible binding poses and then ranked using the HYDE postprocessing scoring function to estimate the interaction energy of the hypothetical Hakai-inhibitor complexes. The first 20 top-ranking molecules were visually inspected and two of them were selected for subsequent experimental validation, namely Hakin-1 and Hakin-5 (Fig.1a). According to our binding mode model, the benzoate moiety present in Hakin-1 would be a surrogate of phosphotyrosine (Fig. 1b, upper panel). The carboxylate group is placed in an extremely favourable region for a negatively charged probe, as estimated by our affinity maps. This region is lined by residues Lys-126, Tyr-176, His-185 and Arg-189, while the phenyl ring would be sandwiched between the guanidinium, side-chains of Arg-174 and Arg-189. The rest of the molecule would be able to establish hydrogen bonds with the side chains of Arg-174 residues from both monomers and also the Gln-170 backbone while maintaining the required shape. Hakin-5 (Fig. 1b, lower panel), despite bearing a carboxylate group and presenting an equivalent number of potential groups for hydrogen bonding interactions, lacks a phenyl ring that could mimic a phosphotyrosine.

### Effect of Hakin-1 inhibitor on Hakai-induced ubiquitination

We first investigated the effect of Hakin-1 inhibitor on the ubiquitination induced by the E3 ubiquitin-ligase Hakai by using culture tumour cells. 293T cells were transfected with Src, Hakai and ubiquitin in presence of Hakin-1 inhibitor or DMSO as control. Hakin-1 strongly reduces the ubiquitination mediated by Hakai in a doses dependent-manner (Fig. 1c) and no effect was seen in Hakai protein levels. However, Hakin-1 did not affect ubiquitination when Hakai was not overexpressed, confirming that Hakin-1 reduces the ubiquitination in a Hakai-dependent manner (Fig. 1d). Moreover, no effect was detected on Hakai-mediated ubiquitination when cells where treated in presence of another identified Hakai inhibitor by virtual screening, Hakin-5, (Fig. 1e), further supporting the specific effect of Hakin-1 on Hakai-induced ubiquitination. Finally, we observed a reduction of Hakai-dependent ubiquitination of E-cadherin complex when cells were treated with Hakin-1 (Fig. 1f). Collectively, our data show that Hakin-1 inhibits Hakai-mediated ubiquitination without affecting Hakai protein levels.

### Hakai inhibition by Hakin-1 activates epithelial differentiation on tumour cells

Next, we studied the effect of Hakai inhibition on the cell viability of cancer cells. For this objective, we generated a dose-response curve by using Hakin inhibitors in several epithelial cells as we previously reported. First, we analysed the cytotoxicity effect on Hakin-1 on HT-29 and LoVo colon tumour cell lines showing an important inhibitory response (Fig. 2a). We extended our studies by using a normal epithelial MDCK cell line that has been extensively used as an in vitro model system to study EMT. As previously showed, Hakai overexpression in MDCK cells (Hakai-MDCK) induced a fibroblastic-like phenotype and a disappearance of E-cadherin-based cell-cell contacts. When treating these cell lines with Hakin-1 or Hakin-5 inhibitor, both clones tested of Hakai-MDCK cell lines were more sensitive to Hakin-1 and Hakin-5 treatment compared to normal epithelial MDCK that were more resistant (Fig. 2b). These results further suggest that Hakin-1 may be particularly effective against cancer cells on which Hakai is overexpressed, as it is seen in human colon cancer, where Hakai is highly increased in colon carcinoma compared to adjacent normal tissues. Given the previously reported role of Hakai on the epithelial-to-mesenchymal transition, we next analysed the effect of Hakin-1 on tumour cell phenotype. By phase contrast, we observed that both Hakin modestly induce an epithelial phenotype on HT-29 and LoVo cells, however, it is important to note that both cell lines already show an epithelial morphology (Fig. 2c). Moreover, we tested the effect of Hakin-1 and Hakin-5 inhibitors on the mesenchymal phenotype of Hakai-transformed MDCK cells. We observed an induction of an epithelial phenotype, increasing cell-cell contacts, accompanied with a reduction of protrusions formation. On the contrary, no effect was observed when treating epithelial MDCK cells with the specific inhibitors (Fig. 2d). Given the reported action of Hakai during EMT, by it molecular action on the E-cadherin ubiquitination, endocytosis and degradation, leading the alteration of cell-cell contacts, we further study the effect of Hakin-1 inhibitor on the reversion of EMT. As shown in figure 3a, by western-blotting we observed that Hakin-1 was able to increase E-cadherin levels in HT-29 cells, while the mesenchymal Vimentin marker was reduced. We also analysed the effect of Hakin-1 on Cortactin, another reported substrate for Hakai, confirming its effect on the increase expression levels. We also confirmed these results by using another epithelial tumour cells, LoVo cells (Fig. 3b). Moreover, as E-cadherin loss at cell-cell contacts is considered a hallmark of the EMT, we studied the expression of E-cadherin by immunofluorescence showing a significant increase E-cadherin levels at cell-cell contacts in HT-29 and LoVo cells (Fig. 3c). However, no effect by western-blotting or immunofluorescence was detected on E-cadherin protein levels when using Hakin-5 (Figure 8). Finally, to test the effect of Hakin-1, mesenchymal Hakai-MDCK cells were also used. Hakai-MDCK cells did not express basal protein levels of E-cadherin, therefore no recovery was detected under Hakin-1 treatment. Taken together, these results show that Hakin-1 induces epithelial differentiation in different tumour epithelial cells, which is accompanied by a reduction of mesenchymal marker *in vivo.*

### Hakin-1 inhibits proliferation, oncogenic potential and invasion in tumour culture cells

We next characterized the effect of Hakin-1 in tumour cell lines using standard proliferation and soft agar colony-forming assays. Given that Hakai affects not only cell-cell contacts but also proliferation in fibroblast and epithelial cells, we decided to determine the possible effect of Hakin-1 in cell proliferation. Hakin-1 reduced cell proliferation in HT29 and LoVo cell lines (Fig. 4a), although no effect was seen using Hakin-5 inhibitor (Fig. 4b), further supporting Hakin-1 antitumor action by its control on cell proliferation. We extended our analysis by using Hakai-MDCK cells compared to normal epithelial MDCK cells. As previously reported, we confirmed that Hakai-transformed MDCK cells strongly increased cell proliferation compared to normal MDCK cell (Fig. 4c). Interestingly, Hakin-1 was able to suppress proliferation in Hakai-MDCK cell while no effect was detected in non-transformed epithelial MDCK cells (Fig. 4c). Hakin-1 also inhibits cell proliferation in other epithelial cells lines such as breast cancer MCF7 cell, prostate cancer PC-3 cells, bladder cancer 5637 cells, liver cancer HepG2 cells and renal cancer ACHN cells (Fig. 9). These results suggest that Hakin-1 may function as antiproliferative agent when high levels of Hakai are expressed, such as it occurs in human adenocarcinoma. By using soft agar colony formation assays, we evaluated the effect of Hakin-1 in HT-29 and Hakai-MDCK tumour cell lines, showing a robust inhibition of colony formation in both cell lines (Fig. 4d). As we previously published, no colony formation was detected while treating MDCK non-transformed cells by Hakin-1. EMT process is characterized by the acquisition of migratory and invasive capabilities. We demonstrated that Hakin-1 strongly reduced cell invasion in LoVo cells (Fig. 5a). Moreover, while no cell invasion was detected in MDCK cells, Hakai overexpression induce the ability to invade in normal epithelial MDCK cells, and in these Hakai-MDCK cells Hakin-1 also decreased cell invasion (Fig. 5b). Finally, given that HT-29 cells were unable to invade under these experimental conditions, we tested Hakin-1 effect in cell motility, showing an important reduction of cell migration under Hakin-1 treatment (Fig. 5c). All these findings support an antitumor effect of Hakin-1 by acting on cell proliferation, oncogenic potential, cell motility and invasion.

### In vivo antitumor effect of Hakin-1 in tumour xenografts

The acquisition of migratory and invasive abilities during EMT are crucial events in the formation of distant metastasis, therefore targeting these events is therefore an ideal approach for cancer treatment. Since we have shown that Hakin-1 effectively inhibits cell proliferation, oncogenic potential and cellular invasion and motility in cell cultures, we decided to study the efficacy of Hakin-1 on this suppression of pre-existing tumours *in vivo.* For this purpose, MDCK and Hakai-MDCK cell were subcutaneously injected into the flank of nude mice. As previously reported, Hakai-MDCK cells formed primary tumours whereas parental MDCK cells were unable to do so. Hakin-1 displayed a potent effect on inhibiting xenograft tumour growth *in vivo* (Fig. 6a). Morphologically xenograft tumour cells exhibits undifferentiated and spindle-shape phenotype, large nucleus and a reduction of the cytoplasm size. This morphology was strongly altered by Hakin-1 treatment, showing an induction of tumour differentiation and an increase in cytoplasm size (Fig. 6b). Moreover, we found tumour-cell infiltration in blood vessels, whereas no infiltration was detected in Hakin-1 treated xenografts tumours (Fig. 6c). Furthermore, by analysing two proliferative markers, Ki67 and the mitotic index, it was shown that Hakin-1 markedly reduce the number of Ki67-positive cells and the mitotic-index (Fig. 6d-e), while no effect on apoptosis was detected (Fig. 10), further underscoring Hakin-1 effect on the inhibition of cell proliferation *in vivo.* We also visualized blood vessels in tumour sections by immunohistochemistry using CD31 angiogenic marker. A strong reduction on the number of blood vessels in Hakin-1 treated xenograft tumours compared to non-treated tumours was quantified indicating its inhibitory effect on angiogenesis (Fig. 6f). Interestingly, no damage was observed in lung and kidney tissues in Hakin-1 treated nude mice, showing a normal morphological structure, supporting that Hakin-1 treatment inhibits tumour growth in nude mice apparently without systemic toxicity (Fig. 11).

### Hakin-1 treatment reduces N-cadherin mesenchymal markers of tumours xenograft and micrometastasis formation in lung in vivo

We further evaluated the *in vivo* effect of Hakin-1 on the reversion of the EMT, as crucial process in tumour progression and cell invasion. First, we confirmed that Hakai protein expression levels were not affected by Hakin-1 action in xenograft tumours of nude mice (Fig. 7a), further supporting previous *in vitro* results where it was shown that Hakin-1 inhibited Hakai activity by its action on its ubiquitin-ligase activity without altering Hakai protein expression (Fig. 1). Given that that E-cadherin was completely disappeared in Hakai-MDCK cells, as expected, E-cadherin was not detected in Hakai-MDCK xenografts tumours in nude mice in presence or absence of Hakin-1 (Fig. 7b). However, when we studied the expression levels of the N-cadherin mesenchymal marker, another hallmark of the EMT, we found that N-cadherin expression was strongly reduced in tumour xenografts treated with Hakin-1 compared to DMSO treated mice (Fig. 7c). These data further support that Hakin-1 inhibits mesenchymal-type tumour cells, by a reduction of N-cadherin mesenchymal marker. Given that the best described target E-cadherin for Hakai was completely absent in Hakai-MDCK mesenchymal tumours, we also extended our study by analysing another described target for Hakai, Cortactin. Cortactin is a cytoskeleton protein and one of the major substrates for Src kinase. Interestingly, Cortactin expression was only detected in the cytoplasm of Hakai-MDCK xenograft tumours and its expression was recovered by Hakin-1 treatment (Fig. 7d), further supporting that Hakin-1 may inhibit the induction of the ubiquitination and degradation of Cortactin by Hakai. To determine whether Hakin-1 may impact on cancer metastasis, lung tissues from nude mice were analysed by H&E staining, however, distant metastasis was not detected under the experimental conditions (Fig. 7e). Therefore, in order to detect the possible presence of micrometastasis, we performed a quantitative PCR by analysing the presence of DNA of Hakai-MDCK in lung. For this purpose, HA-tagged Hakai present in Hakai-MDCK cells was measured by using two different specific primers: one designed for HA epitope and the second primer for Hakai. Hakin-1 caused a significant reduction of micrometastasis detected in lung of the Hakai-MDCK xenograft mice compared to the control DMSO treated mice, while no detection was found in lung of MDCK-injected mice (Fig. 7f). This result underscore that Hakin-1 inhibits the metastasis to lung *in vivo.*

### Effect of Hakin-1 analogues on cytotoxicity and on Hakai-induced ubiquitination

Next, we studied the effect of the selected analogues on HT29 colon cancer cells. First, we analysed cytotoxicity effect of the following analogues: Ketophenyls A-1, A-7, A-8 and A-9; ketoheteroaryls: A-23 and A-25; Cyclic amides: A-10 and A-16 and Bencylamide A-6.1 on HT-29 colon tumour cell lines. It is shown an important inhibitory response of the Ketophenyls A-7, A-8, A-9 and ketoheteroaryls: A-23, A-25, however no cytotoxic effect was detected by the action of Ketophenyls A-1, Cyclic amides: A-10 and A-16 and Bencylamide A-6.1 (Fig. 12). Then, we investigated the effect of specific selected analogues on the ubiquitination induced by the E3 ubiquitin-ligase Hakai by using culture tumour cells. 293T cells were transfected with Src, Hakai and ubiquitin in presence of the specific analogue inhibitors or DMSO as control. An important reduction of the ubiquitination mediated by Hakai in a doses dependent-manner was observed by using Ketophenyls A-7 analogue (Fig. 13), showing an effect on the reduction of Hakai activity without affecting Hakai protein levels. On the other hand, analogue Ketophenyls A-9 reduced Hakai-mediated ubiquitination but reducing Hakai protein levels at the concentration tested. Moreover, cyclic amides: A-10 and A-16 modestly reduced the ubiquitination mediated by Hakai, without affecting protein levels. Finally, it was also detected an inhibitory effect of Hakai activity by the action ketoheteroaryls A-23, at a low concentration.

## Claims

1. A compound of formula (I) 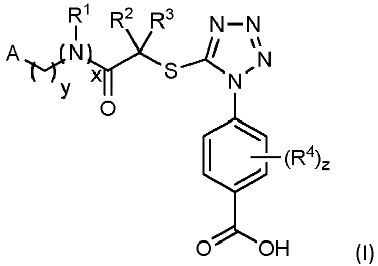 wherein:
- A represents a group selected from aryl, heteroaryl and cyclic amides optionally substituted by 1 or 2 groups that are independently selected from:
i) halogen atom, -CN, -N(R^{a})R^{b}, -OR^{a}, -C(=O)R^{a}, -C(=O)OR^{a}, -C(=O)N(R^{a})R^{b}, -OC(=O)-R^{a}, -N(R^{c})C(=O)R^{b}, -NR^{c}SO₂R^{a}, -SO₂N(R^{a})R^{b}, -SR^{a}, -S(O)R^{a}, -S(O)₂R^{a};
j) linear or branched C₁-C₆ alkyl, optionally substituted by 1, 2 or 3 halogen atoms;
k) C₃-C₆ cycloalkyl which optionally contains 1 or 2 heteroatoms selected from O, S and N, and which ring is optionally substituted by C₁-C₃ alkyl;
I) phenyl or C₅-C₆ heteroaryl each optionally substituted by halogen atom, cyano group, C₁-C₃ alkyl or cyclopropyl;
- each R^{a}, R^{b} and R^{c} independently represents:
i) hydrogen atom,
j) linear or branched C₁-C₁₂ alkyl, C₃-C₆ cycloalkyl and C₄-C₆ heterocycloalkyl, which are optionally substituted by 1, 2 or 3 substituents selected from a carbonyl group, halogen atom, hydroxy, phenyl, C₃-C₆ cycloalkyl, linear or branched C₁-C₆ alkoxy, amino, alkylamino, dialkylamino, linear or branched C₁-C₆ alkylcarbonyl,
k) phenyl or C₅-C₆ heteroaryl group, which are optionally substituted by 1, 2 or 3 substituents selected from halogen atom, cyano group, linear or branched C₁-C₆ alkyl, linear or branched C₁-C₆ haloalkyl, hydroxy, linear or branched C1-C6 alkoxy, amino, alkylamino, dialkylamino;
l) R^{a} and R^{b} form together with the nitrogen atom to which they are attached, a 3- to 8 membered ring which optionally contains a further heteroatom selected from O, N and S, and which ring is optionally substituted by 1, 2 or 3 substituents selected from carbonyl group, linear or branched C₁-C₆ alkyl, linear or branched C₁-C₆ haloalkyl, linear or branched C₁-C₆ alkylcarbonyl;
- x and y are integers independently selected from 0 and 1;
- R¹ represents a group selected from hydrogen, cyclopropyl or linear or branched C₁-C₆ alkyl, wherein said alkyl is optionally substituted by 1, 2 or 3 halogen atoms;
- when y is 0, then 2 or 3 carbon atoms of R¹ can form a 5- or 6-membered ring together with the neighboring nitrogen atom and the 2 adjacent carbon atoms of the aromatic ring to which the nitrogen is attached;
- each R² and R³ independently represent a group selected from hydrogen, cyclopropyl or linear or branched C₁-C₆ alkyl;
- R² and R³ can form a 3-or 4-membered spiro ring together with the carbon atom to which they are both attached;
- z is an integer selected from 0, 1, 2 or 3;
- R⁴ represents a group selected from -CN, cyclopropyl or linear or branched C₁-C₆ alkyl, said alkyl is optionally substituted by 1, 2 or 3 halogen atoms; wherein the group R⁴, if present, replaces the hydrogen atom of one of the groups CH present in the phenyl ring to which R⁴ is attached;
and pharmaceutically acceptable salts thereof;
for use in the treatment of cancer.

2. The compound for use according to claim 1, wherein the cancer is a carcinoma.

3. The compound for use according to claim 2, wherein the cancer is a carcinoma selected from the list consisting of tumors arising from epithelial layers of the gastrointestinal track including month (oral cancer), esophagus, stomach, and small and large intestines (such as rectal or colon cancer), skin cancer, mammary gland (breast cancer), pancreas cancer, lung cancer, head and neck cancer, liver cancer, ovary cancer, cervix cancer, uterus cancer, gallbladder cancer, penile cancer, and urinary bladder cancer (such as renal, prostate or bladder cancer).

4. The compound for use according to any of claims 1 to 3, wherein each R² and R³ independently represents a group selected from cyclopropyl or a linear or branched C₁-C₆ alkyl.

5. The compound for use according to any of claims 1 to 3, wherein R² and R³ form a 3-or 4-membered spiro ring together with the carbon atom to which they are both attached.

6. The compound for use according to any of claims 1 to 3, wherein z is an integer selected from 1, 2 or 3; wherein R⁴ represents a group selected from -CN, cyclopropyl or a linear or branched C₁-C₆ alkyl, wherein said alkyl is optionally substituted by 1, 2 or 3 halogen atoms; and wherein group R⁴ replaces the hydrogen atom of one of the groups CH present in the phenyl ring to which R⁴ is attached.

7. The compound for use according to any of claims 1 to 3, wherein one or both of the integers x and y equal 1 and A represents a group selected from aryl, heteroaryl and cyclic amides optionally substituted by 1 or 2 groups that are independently selected from halogen atom, -CN, -N(Ra)Rb, -ORa, -C(=O)Ra, -C(=O)ORa, -C(=O)N(Ra)Rb, -OC(=O)-Ra, -N(Rc)C(=O)Rb, -NRcSO2Ra, -SO2N(Ra)Rb, -SRa, -S(O)Ra, -S(O)2Ra, linear or branched C1-C6 alkyl, wherein said alkyl is optionally substituted by 1, 2 or 3 halogen atoms; C3-C6 cycloalkyl which optionally contains 1 or 2 heteroatoms selected from O, S and N, and which ring is optionally substituted by C1-C3 alkyl; phenyl or C5-C6 heteroaryl each optionally substituted by halogen atom, C1-C3 alkyl or cyclopropyl.

8. The compound for use according to any of claims 1 to 3, wherein the compound is selected from the list consisting of any of the following compounds:

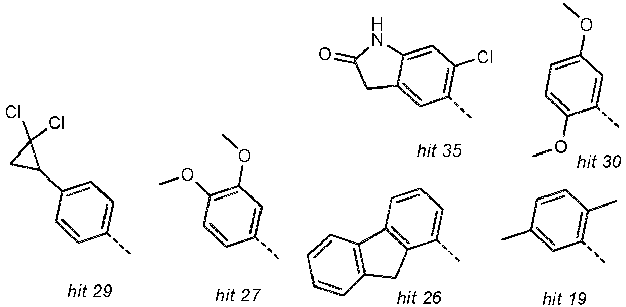

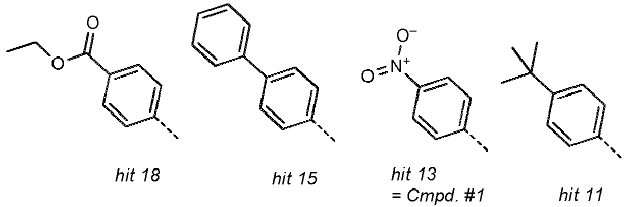

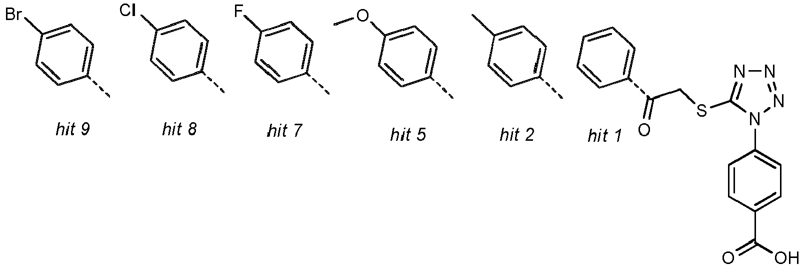

9. The compound for use according to any of claims 1 to 3, wherein the compound is 4-(5-((2-(4-nitrophenyl)-2-oxoethyl)thio)-1H-tetrazol-1-yl)benzoic acid.

10. The compound for use according to any of claims 1 to 3, wherein the compound is selected from the list consisting of any of the following compounds: 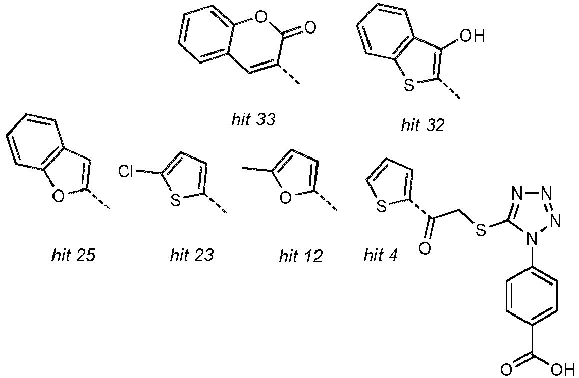 Or

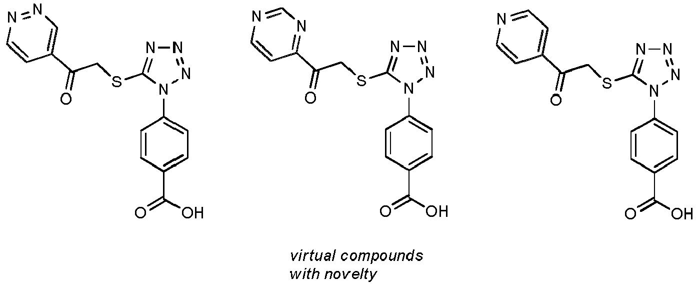

11. The compound for use according to any of claims 1 to 3, wherein the compound is selected from the list consisting of any of the following compounds:

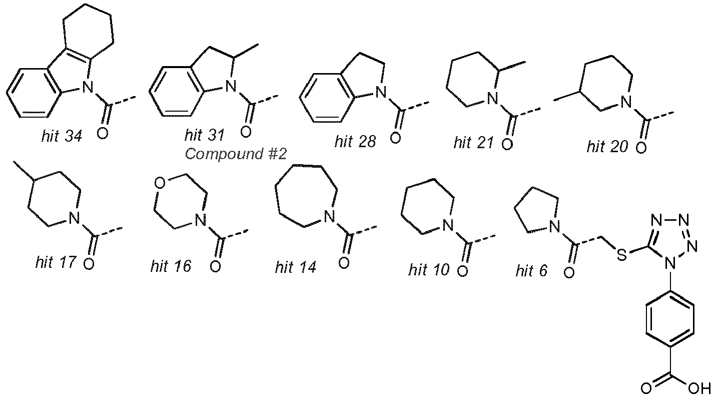

12. The compound for use according to any of claims 1 to 3, wherein the compound is 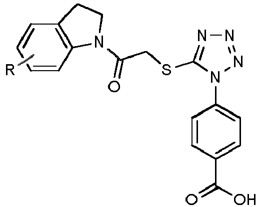 wherein R represents a group selected from hydrogen, cyclopropyl or linear or branched C1-C6 alkyl, wherein said alkyl is optionally substituted by 1, 2 or 3 halogen atoms.

13. The compound for use according to any of claims 1 to 3, wherein the compound is 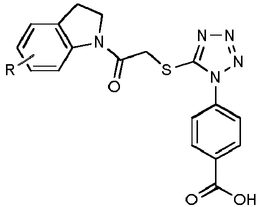 wherein R is a methyl group.

14. The compound for use according to any of claims 1 to 3, wherein the compound is selected from the list consisting of any of the following compounds:
